# FASCICULE DE BREVET EUROPEEN

(11) **EP 0 577 458 B1**
(45) Date de publication et mention de la délivrance du brevet: **29.09.2004**
(21) Numéro de dépôt: 93401538.9
(22) Date de dépôt: 16.06.1993
(51) Int. Cl.: C12N 15/49, C12N 15/70, C12Q 1/68, A61K 39/21, G01N 33/53, G01N 33/68

(54) **Séquences nucléotidiques et peptidiques issues de la souche Wo du VIF et applications desdites séquences au diagnostic et à la prévention de l'immunodéficience féline**
Nukleotid- und Peptidsequenzen des Immunschwächevirus der Katze, Isolat WO und Anwendungen der Sequenzen in der Diagnostik und zur Verhinderung der Infektion
Nucleotide and Peptide sequences of the FIV isolate WO and their uses in diagnostic and prevention of the feline immunodeficiency virus infection

(30) Priorité: 16.06.1992 FR 9207257; 16.06.1992 FR 9207258; 23.11.1992 FR 9214026
(43) Date de publication de la demande: 05.01.1994
(73) Titulaire: CENTRE NATIONAL DE LA RECHERCHE SCIENTIFIQUE, 75016 Paris Cédex (FR)
(72) Inventeur: Pancino, Gianfranco, F-75019 Paris (FR); Chappey, Colombe, F-77590 Bois le Roi (FR); Hurtrel, Bruno, F-44240 La Chapelle sur Erdre (FR); Moraillon, Anne, F-94210 La Varenne Saint Hilaire (FR); Klatzmann, David, F-75103 Paris (FR); Sonigo, Pierre, F-75015 Paris (FR); Saurin, William, F-75015 Paris (FR); Avrameas, Alexandre, F-94400 Vitry sur Seine (FR); Strosberg, Arthur Donny, F-75015 Paris (FR)
(74) Mandataire: Ores, Irène

(56) Documents cités:
- FR-A- 2 669 338
- MOLECULAR IMMUNOLOGY vol. 29, no. 5, Mai 1992, BPCC WHEATONS, EXETER,GB; pages 565 - 572 A. AVRAMEAS ET AL. 'Localization of three epitopes of the ENV protein of feline immunodeficiency virus'
- VETERINARY MICROBIOLOGY vol. 31, no. 1, Avril 1992, ELSEVIER, AMSTERDAM, NL; pages 41 - 54 A. MORAILLON ET AL. 'In vitro properties and experimental pathogenic effect of three strains of feline immunodeficiency viruses (FIV) isolated from cats with terminal disease'
- PATENT ABSTRACTS OF JAPAN vol. 16, no. 387 (C-975)(5430) 18 Août 1992
- ARCH. VIROL. vol. 123, no. 1-2, 11 Mars 1992, SPRINGER VERLAG, VIENNE, AUSTRICHE; pages 29 - 45 N. MAKI ET AL. 'Molecular characterization and heterogeneity of feline immunodeficiency virus'
- JOURNAL OF VIROLOGY vol. 67, no. 2, Février 1993, AM. SOC. MICROBIOL., BALTIMORE, US; pages 664 - 672 G. PANCINO ET AL. 'B epitopes and selection pressures in feline immunodeficiency virus envelope glycoproteins'
- VIROLOGY vol. 192, no. 2, Février 1993, ACADEMIC PRESS, NEW YORK, US; pages 659 - 662 G. PANCINO ET AL. 'Structure and variations of feline immunodeficiency virus envelope glycoproteins'

## Description

La présente invention est relative à des peptides et à leurs fragments, spécifiques du virus de l'immunodéficience féline (VIF) ainsi qu'à l'utilisation desdits fragments comme réactif de diagnostic et comme agent induisant une réponse immune (cellulaire et/ou production d'anticorps), notamment pour la prévention de l'immunodéficience féline.

La présente invention est également relative aux séquences nucléotidiques codant pour lesdits peptides.

L'immunodéficience féline est due à un lentivirus, le virus de l'immunodéficience féline (VIF), qui présente une structure génétique similaire à celle des lentivirus des primates (VIH et VIS).

L'immunodéficience féline pose un problème important de santé vétérinaire, dans la mesure où un nombre important de chats infectés par le VIF a été décelé aux Etats-Unis, au Japon et en Europe (5 à 25 % des animaux).

A l'heure actuelle, l'immunodéficience féline est essentiellement diagnostiquée lors de l'apparition des signes cliniques (lymphadénopathie généralisée et survenue d'infections opportunistes), alors qu'un diagnostic très précoce aurait des avantages importants tant dans le traitement que dans la prévention de cette maladie.

Plusieurs isolats viraux indépendants ont été mis en évidence à travers le monde et un certain nombre de travaux pour mettre en évidence la structure des souches isolées ont été réalisés, notamment en ce qui concerne la souche américaine *Petaluma* [R.L. TALBOTT et al. (Natl. Acad. Sci. USA, 1989, 86, 5743-5747) ; T.R. PHILIPPS et al. (J. Virol., 1990, 64, 10, 4605-4613)], les souches japonaises (souches TM1 et TM2) [T. MIYAZAWA et al. (Arch. Virol., 1989, 108, 59-68)] ou les isolats suisses (FIVZ1 et FIVZ2) [S. MORIKAWA et al., (Virus Research, 1991, 21, 53-63)].

Les séquences nucléotidiques de trois clones proviraux, dérivés des isolats de VIF américains (souche *Petaluma*) ont été décrites (clones FIV34TF10, FIV14 et isolat PPR) [R.A. OLMSTED et al., (Proc. Natl. Acad. Sci. USA, 1989, 86, 2448-2452) ; T.R. PHILIPPS et al., 1990 ; R.L. TALBOTT et al., (Froc. Natl. Acad. Sci. USA, 1989, 86, 5743-5747) et comparées aux deux isolats suisses (S. MORIKAWA et al.). Cette comparaison a conduit S. MORIKAWA et al. à préciser la présence de certaines régions conservées et de certaines régions variables dans le gène *env* du VIF.

Des souches françaises ont également été isolées (souches Wo et Me) [MORAILLON A. et al., 1992, Vet. Mic. , 31, 41-45, *In vitro properties and experimental pathogenic effect of three feline immunodeficiency viruses isolated from cats with terminal diseases*].

Les protéines d'enveloppe du VIF sont considérées comme étant au centre de la relation hôte-virus ; leur étude est donc essentielle pour comprendre l'interaction du virus avec le système immunitaire (épitopes de neutralisations, épitopes B et T) et avec les cellules-cibles de l'infection, et mettre au point aussi bien des réactifs de diagnostic performants que des vaccins efficaces, constitués de protéines virales à action immunogène et protectrice, vis-à-vis de l'ensemble des souches de VIF.

La protéine env du VIF peut fournir, après clivage, deux fragments glycoprotéiques, dénommés SU (glycoprotéine de surface) et TM (glycoprotéine transmembranaire).

Il a été montré que des fragments peptidiques issus d'une protéine env du VIF contiennent des déterminants majeurs de la protection (Demande de Brevet français n° 2 669 338 ou Demande Internationale PCT WO 92/09632 au nom de la Demanderesse).

Poursuivant ses travaux, la Demanderesse a trouvé :
- d'une part, qu'en vue de mettre au point des outils de diagnostic plus performants et des moyens de production de vaccins spécifiquement protecteurs, il était important de sélectionner, à partir de ces souches, des séquences aptes à être performantes dans le diagnostic différentiel précoce de l'immunodéficience féline et aptes à produire des produits dérivés par génie génétique, à fin de vaccination, ou à choisir une immunothérapie adaptée à la souche infectante,
- et d'autre part, qu'il était nécessaire, pour optimiser aussi bien le diagnostic que la prévention (vaccins sous-unités), de sélectionner des peptides viraux de petites tailles, particulièrement adaptés au diagnostic et à la prévention et permettant de former un pool de réactifs pour la détection de groupe (ensemble des souches de VIF) et/ou la détection de type (détection sélective de certaines souches).

La présente invention s'est en conséquence donné pour but de pourvoir, d'une part, à des séquences issues du VIF et à leurs fragments sélectionnés notamment pour leur spécificité de souche, et, d'autre part, à une composition à pouvoir immunogène et/ou protecteur, spécifique du virus de l'immunodéficience féline, obtenue en exprimant des fragments sélectionnés pour leur capacité neutralisante et/ou la présence d'épitopes de reconnaissance de groupe et/ou de type ; l'obtention par génie génétique ou synthèse chimique de tels peptides spécifiques a pour avantage de résoudre le problème de production de virus et d'obtenir directement les peptides désirés.

Des séquences nucléotidiques issues de l'ARN génomique du virus de l'immunodéficience féline (VIF), comprennent au moins un fragment d'un gène présentant des zones hypervariables, choisi dans le groupe constitué par le gène codant pour la protéine env de la souche Wo du VIF et le gène codant pour la protéine gag de la souche Wo du VIF.

Conformément à ladite séquence, celle-ci comprend la séquence en nucléotides et la séquence déduite en amino-acides de formule I ci-après, qui correspond au gène env du VIF, souche Wo :

1/1                                                    31/11
atg gca gaa ggg ttt gca gct aat aga caa tgg ata ggg cca
Met ala glu gly phe ala ala asn arg gln trp ile gly pro
                              61/21
gaa gaa gct gaa gag cta tta gat ttt gat ata gca ata caa
glu glu ala glu glu leu leu asp phe asp ile ala ile gln
          91/31                                        121/41
atg aat gaa gaa ggg cca cta aat cca gga gta aac cca ttt
met asn glu glu gly pro leu asn pro gly val asn pro phe
                                        151/51
agg gta cct gga ata aca gaa gca gaa aag caa gaa tat tgt
arg val pro gly ile thr glu ala glu lys gln glu tyr cys
                    181/61
aac ata tta caa ccc aag tta caa gat cta aag ggg aaa att
asn ile leu gln pro lys leu gln asp leu lys gly lys ile
211/71                                             241/81
caa gag gta aaa ctg gaa gaa gga aat gca ggt aag ttt aga
gln glu val lys leu glu glu gly asn ala gly lys phe arg
                              271/91
aga gca aga ttt tta aga tac tct gat gaa aca gta ttg tcc
arg ala arg phe leu arg tyr ser asp glu thr val leu ser
          301/101                                      331/111
cta att cac ttg ttc ata gga tat tgt cca cat tta tgc aga
leu ile his leu phe ile gly tyr cys pro his leu cys arg
                                        361/121
cga cat gaa tta gga tct tta aga cat gac ata gat ata gaa
arg his glu leu gly ser leu arg his asp ile asp ile glu
                    391/131
gca ctt caa gaa gag cgt tat aat gat aga gag aag gga ata
ala leu gln glu glu arg tyr asn asp arg glu lys gly ile
421/141                                            451/151
act gac aat ata aaa tat ggt aaa cga tgc tta ata gga aca
thr asp asn ile lys tyr gly lys arg cys leu ile gly thr
                              481/161
gcg gtt ttg tac ctg ctt cta tct ttg gga ata ata ata cat
ala val leu tyr leu leu leu ser leu gly ile ile ile his
          511/171                                      541/181
aca tgc aag gct cag gta gta tgg aga ctt cca cca tta gta
thr cys lys ala gln val val trp arg leu pro pro leu val
                                        571/191
gtc cca gta gaa gaa tca gaa ata att ttt tgg gat tgt tgg
val pro val glu glu ser glu ile ile phe trp asp cys trp

601/201
gca cca gag gaa ccc gcc tgt cag gac ttt ctt ggg gca atg
ala pro glu glu pro ala cys gln asp phe leu gly ala met
631/211                                         661/221
ata cat cta aaa gct agt aca aat ata agt ata caa gag gga
ile his leu lys ala ser thr asn ile ser ile gln glu gly
                        691/231
cct acc ctg ggg aat tgg gct aga gaa ata tgg gga aca tta
pro thr leu gly asn trp ala arg glu ile trp gly thr leu
        721/241                                 751/251
ttc aaa aag gct acc aga caa tgt aga aga ggt aga ata tgg
phe lys lys ala thr arg gln cys arg arg gly arg ile trp
                                781/261
aga aga tgg aat gaa act ata aca gga ccg tta gga tgt gct
arg arg trp asn glu thr ile thr gly pro leu gly cys ala
                811/271
aat aat aca tgt tat aat atc tca gta ata gta cct gat tat
asn asn thr cys tyr asn ile ser val ile val pro asp tyr
841/281                                         871/291
caa tgt tat cta gat aga gta gat act tgg tta caa ggg aaa
gln cys tyr leu asp arg val asp thr trp leu gln gly lys
                        901/301
gta aat ata tca tta tgt cta aca gga gga aaa atg tta tat
val asn ile ser leu cys leu thr gly gly lys met leu tyr
        931/311                                 961/321
aat aaa gag aca aaa caa tta agc tat tgt aca gac cca tta
asn lys glu thr lys gln leu ser tyr cys thr asp pro leu
                                991/331
caa atc cca ctg atc aat tat aca ttt gga cct aat caa aca
gln ile pro leu ile asn tyr thr phe gly pro asn gln thr
                1021/341
tgt atg tgg aac act tca cag atc caa gac cct gag ata cca
cys met trp asn thr ser gln ile gln asp pro glu ile pro
1051/351                                        1081/361
aaa tgt gga tgg tgg aat caa aac gcc tat tat aat agt tgt
lys cys gly trp trp asn gln asn ala tyr tyr asn ser cys
                        1111/371
aga tgg gaa cac aca gat gta cag ttt caa tgt caa agg aca
arg trp glu his thr asp val gln phe gln cys gln arg thr
        1141/381                                1171/391
caa agt caa cca gga tca tgg att aga gca atc tcg tca tgg
gln ser gln pro gly ser trp ile arg ala ile ser ser trp
                                1201/401
aaa caa agg aat aga tgg gaa tgg aga cca gac ttt gaa agt
lys gln arg asn arg trp glu trp arg pro asp phe glu ser
                1231/411
gaa aaa gta aaa gta tct cta cag tgt aat agc aca aaa aac
glu lys val lys val ser leu gln cys asn ser thr lys asn
1261/421                                        1291/431
cta acc ttt gca atg aga agt tca gga gat tat gga gaa gta
leu thr phe ala met arg ser ser gly asp tyr gly glu val
                        1321/441
aca gga gct tgg ata gag ttt gga tgt cat agg aca aaa tca
thr gly ala trp ile glu phe gly cys his arg thr lys ser

1351/451                                                    1381/461
aaa tat cat act gaa gca agg ttt aga att aga tgt aga tgg
lys tyr his thr glu ala arg phe arg ile arg cys arg trp
                                        1411/471
aat gta ggg gat aat acc tca ctc att gat aca tgt ggg gaa
asn val gly asp asn thr ser leu ile asp thr cys gly glu
                    1441/481
act caa aat gtt tca aga gca aat cct gta gat tgt acc atg
thr gln asn val ser arg ala asn pro val asp cys thr met
1471/491                                                1501/501
tat gca aat aga atg tat aac tgt tcc tta caa aac ggg ttt
tyr ala asn arg met tyr asn cys ser leu gln asn gly phe
                              1531/511
act atg aag gta gat gac ctt atc atg cat ttt aat aag aca
thr met lys val asp asp leu ile met his phe asn lys thr
        1561/521                                        1591/531
aaa gct gta gag atg tat aac att gct gga aat tgg tct tgt
lys ala val glu met tyr asn ile ala gly asn trp ser cys
                                        1621/541
aaa tct gac ttg cca cca aca tgg ggg tat atg aat tgt aat
lys ser asp leu pro pro thr trp gly tyr met asn cys asn
                    1651/551
tgt aca aat agt aca aat agt ggt act ggt ata agg atg gca
cys thr asn ser thr asn ser gly thr gly ile arg met ala
1681/561                                                1711/571
tgt ccc aga aat caa ggc atc tta aga aat tgg tat aac cca
cys pro arg asn gln gly ile leu arg asn trp tyr asn pro
                              1741/581
gta gca gga tta aga caa tcc tta gaa aag tat caa gtt gta
val ala gly leu arg gln ser leu glu lys tyr gln val val
        1771/591                                        1801/601
aaa caa cca gat tat tta gtg gta cca ggg gaa gtc atg gaa
lys gln pro asp tyr leu val val pro gly glu val met glu
                                        1831/611
tat aaa cct aga agg aaa aga gca gct att cat gtt atg tta
tyr lys pro arg arg lys arg ala ala ile his val met leu
                    1861/621
gct ctt gca aca gta tta tct atg gct gga gca ggg acg ggg
ala leu ala thr val leu ser met ala gly ala gly thr gly
1891/631                                                1921/641
gct act gct ata ggg atg gta aca caa tat caa caa gtg ctg
ala thr ala ile gly met val thr gln tyr gln gln val leu
                              1951/651
gca act cat caa gaa gct ata gaa aag gtg act gaa gcc tta
ala thr his gln glu ala ile glu lys val thr glu ala leu
        1981/661                                        2011/671
aag ata aac aac tta aga tta gtt aca tta gag cat caa gta
lys ile asn asn leu arg leu val thr leu glu his gln val
                                        2041/681
cta gta ata gga tta aaa gta gaa gct atg gaa aaa ttt tta
leu val ile gly leu lys val glu ala met glu lys phe leu
                    2071/691
tat aca gct ttc gct atg caa gaa tta gga tgt aat caa aat
tyr thr ala phe ala met gln glu leu gly cys asn gln asn

2101/701 2131/711
caa ttc ttc tgt aaa gtc cct tct gca ttg tgg gaa cgg tat
gln phe phe cys lys val pro ser ala leu trp glu arg tyr
2161/721
aat atg act ata aat caa aca ata tgg aat cat gga aat ata
asn met thr ile asn gln thr ile trp asn his gly asn ile
2191/731 2221/741
act ttg ggg gaa tgg tat aac caa aca aaa gat tta caa caa
thr leu gly glu trp tyr asn gln thr lys asp leu gln gln
2251/751
agg ttt tat gaa ata ata atg gac ata gaa caa aat aat gta
arg phe tyr glu ile ile met asp ile glu gln asn asn val
2281/761
caa ggg aaa aaa ggg cta caa caa ctc cag gag tgg gaa gat
gln gly lys lys gly leu gln gln leu gln glu trp glu asp
2311/771 2341/781
tgg gta gga tgg ata gga aat att cca caa tat tta aag gga
trp val gly trp ile gly asn ile pro gln tyr leu lys gly
2371/791
cta ttg gga ggt atc ttg gga ata gga tta gga atg tta tta
leu leu gly gly ile leu gly ile gly leu gly met leu leu
2401/801 2431/811
ttg atc tta tgt tta cct aca ttg gtt gat tgt ata aga aat
leu ile leu cys leu pro thr leu val asp cys ile arg asn
2461/821
tgt atc cac aag ata ctg gga tac aca gta att gca atg cct
cys ile his lys ile leu gly tyr thr val ile ala met pro
2491/831
gaa gta gaa gaa gaa gaa ata caa cca caa atg gaa ttg agg
glu val glu glu glu glu ile gln pro gln met glu leu arg
2521/841 2551/851
aga aat ggt agg caa tgt ggc atg tct gaa aaa gag gag gaa
arg asn gly arg gln cys gly met ser glu lys glu glu glu

tga tga
OPA OPA (I)

Une telle séquence correspond à l'ADNc de l'ARNm de la protéine env de la souche Wo du VIF.

La présente invention a pour objet un peptide issu de la protéine Env du VIF de souche Wo, qui constitue un épitope conservé des protéines du VIF, caractérisé en ce qu'il est choisi dans le groupe constitué :
- du fragment de 45 aminoacides, dénommé TM3, et correspondant aux positions 744-788 de la séquence de formule I ;
- du fragment de séquence Gln⁷⁶³-Gln-Leu-Gln-Glu-Trp-Glu-Asp⁷⁷⁰ (peptide dénommé P241), correspondant aux positions 763-770 de la séquence de formule I ; et
- du fragment de séquence Val⁷⁷²-Gly-Trp-Ile-Gly-Asn-Ile-Pro⁷⁷⁹ (peptide dénommé P242), correspondant aux positions 772-779 de la séquence de formule I.

Ledit peptide est notamment reconnu par les anticorps dirigés contre au moins une souche différente de celle dont provient ledit peptide et/ou par les anticorps produits par des chats infectés naturellement et en ce qu'il présente une capacité d'induction d'une réponse immune cellulaire et/ou de production d'anticorps protecteurs vis-à-vis d'au moins une souche de VIF, chez des chats non infectés.

Le fragment incluant un segment de 45 amino-acides, dénommé TM3, correspond aux positions 744-788 de la séquence de formule II ci-après, lequel segment contient un épitope incluant la séquence Gln⁷⁶⁴-Leu-Gln-Glu-Trp-Glu-Asp-Trp-Val-Gly-Trp-Ile-Gly-Asn-Ile⁷⁷⁸ ou la séquence Gln⁷⁶³-Gln-Leu-Gln-Glu-Trp-Glu-Asp⁷⁷⁰ (peptide dénommé P241) ou la séquence Val⁷⁷²-Gly-Trp-Ile-Gly-Asn-Ile-Pro⁷⁷⁹ (peptide dénommé P242).

Le peptide qui correspond à la protéine Env du VIF, souche Wo, présente la séquence suivante :

Met-Ala-Glu-Gly-Phe-Ala-Ala-Asn-Arg-Gln-Trp-Ile-Gly-Phe-
Glu-Glu-Ala-Glu-Glu-Leu-Leu-Asp-Phe-Asp-Ile-Ala-Ile-Gln-
Met-Asn-Glu-Glu-Gly-Pro-Leu-Asn-Pro-Gly-Val-Asn-Pro-Phe-
Arg-Val-Pro-Gly-Ile-Thr-Glu-Ala-Glu-Lys-Gln-Glu-Tyr-Cys*-
Asn-Ile-Leu59-Gln-Pro-Lys-Leu-Gln-Asp-Leu-Lys-Gly-Lys-
Ile-Gln-Glu-Val-Lys-Leu-Glu-Glu-Gly-Asn-Ala-Gly-Lys-Phe-
Arg-Arg-Ala-Arg-Phe-Leu-Arg-Tyr-Ser-Asp-Glu-Thr-Val-Leu-
Ser-Leu-Ile-His101-Leu-Phe-Ile-Gly-Tyr-Cys-Pro-His109-
Leu-Cys-Arg-Arg-His114-Glu-Leu-Gly-Ser-Leu119-Arg-His121-
Asp-Ile-Asp-Ile-Glu-Ala-Leu-Gln-Glu-Glu-Arg-Tyr-Asn-Asp-
Arg-Glu-Lys-Gly-Ile-Thr-Asp-Asn-Ile-Lys-Tyr-Gly-Lys-Arg-
Cys*-Leu-Ile-Gly-Thr-Ala-Val-Leu-Tyr-Leu-Leu-Leu-Ser-Leu-
Gly-Ile-Ile-Ile-His168-Thr-Cys-Lys-Ala-Gln-Val-Val-Trp-
Arg177-Leu-Pro-Pro-Leu-Val-Val-Pro-Val-Glu-Glu-Ser-Glu-
Ile-Ile-Phe-Trp-Asp-Cys*-Trp-Ala-Pro-Glu-Glu-Pro-Ala-

Cys*-Gln-Asp-Phe-Leu-Gly-Ala-Met-Ile-His-Leu-Lys-Ala-Ser-
Thr-Asn-Ile-Ser-Ile-Gln-Glu-Gly-Pro-Thr-Leu-Gly-Asn-Trp-
Ala-Arg-Glu-Ile-Trp-Gly-Thr237-Leu-Phe-Lys-Lys-Ala-Thr-
Arg-Gln-Cys*-Arg-Arg-Gly-Arg-Ile-Trp-Arg-Arg-Trp-Asn-Glu-
Thr-Ile-Thr-Gly-Pro-Leu-Gly-Cys*-Ala-Asn-Asn-Thr-Cys*-
Tyr-Asn-Ile-Ser-Val-Ile-Val-Pro-Asp-Tyr-Gln-Cys*-Tyr-Leu-
Asp-Arg-Val-Asp-Thr-Trp-Leu-Gln-Gly-Lys-Val-Asn-Ile297-
Ser-Leu-Cys*-Leu-Thr-Gly-Gly-Lys-Met-Leu-Tyr-Asn-Lys-Glu-
Thr-Lys-Gln-Leu-Ser-Tyr-Cys*-Thr-Asp-Pro-Leu-Gln-Ile-Pro-
Leu-Ile-Asn-Tyr-Phe-Gly-Pro-Asn-Gln-Thr-Cys*-Met-Trp-Asn-
Thr-Ser-Gln-Ile-Gln-Asp-Pro-Glu-Ile-Pro-Lys-Cys*-Gly-Gly-
Asn-Gln357-Asn-Ala-Tyr-Tyr-Asn-Ser-Cys*-Arg-Trp-Glu-His-
Thr-Asp-Val-Gln-Phe-Gln-Cys*-Gln-Arg-Thr-Gln-Ser-Gln-Pro-
Gly-Ser-Trp-Ile-Arg-Ala-Ile-Ser-Ser-Trp-Lys-Gln-Arg-Asn-
Arg-Trp-Glu-Trp-Arg-Pro-Asp-Phe-Glu-Ser-Glu-Lys-Val-Lys-
Val-Ser-Leu-Gln-Cys*-Asn-Ser417-Thr-Lys-Asn-Leu-Thr-Phe-
Ala-Met-Arg-Ser-Ser-Gly-Asp-Tyr-Gly-Glu-Val-Thr-Gly-Ala-
Trp-Ile-Glu-Phe-Gly-Cys*-His-Arg-Thr-Lys-Ser-Lys-Tyr-His-
Thr-Glu-Ala-Arg-Phe-Arg-Ile-Arg-Cys*-Arg-Trp-Asn-Val-Gly-
Asp-Asn-Thr-Ser-Leu-Ile-Asp-Thr-Cys*-Gly-Glu-Thr477-Gln-
Asn-Val-Ser-Arg-Ala-Asn-Pro-Val-Asp-Cys*-Thr-Met-Tyr-Ala-
Asn-Arg-Met-Tyr-Asn-Cys*-Ser-Leu-Gln-Asn-Gly-Phe-Thr-Met-
Lys-Val-Asp-Asp-Leu-Ile-Met-His-Phe-Asn-Lys-Thr-Lys-Ala-
Val-Glu-Met-Tyr-Asn-Ile-Ala-Gly-Asn-Trp-Ser-Cys*-Lys-Ser-
Asp-Leu-Pro537-Pro-Thr-Trp-Gly-Tyr-Met-Asn-Cys*-Asn-Cys*-
Thr-Asn-Ser-Thr-Asn-Ser-Gly-Thr-Gly-Ile-Arg-Met-Ala-Cys*-
Pro-Arg-Asn-Gln-Gly-Ile-Leu-Arg-Asn-Trp-Tyr-Asn-Pro-Val-
Ala-Gly-Leu-Arg-Gln-Ser-Leu-Glu-Lys-Tyr-Gln-Val-Val-Lys-
Gln-Pro-Asp-Tyr-Leu-Val595-Val-Pro-Gly-Glu-Val-Met-Glu-
Tyr-Lys-Pro-Arg-Arg-Lys-Arg-Ala-Ala-Ile-His-Val-Met-Leu-
Ala-Leu-Ala-Thr-Val-Leu-Ser-Met-Ala-Gly-Ala-Gly-Thr-Gly-
Ala-Thr-Ala-Ile-Gly-Met-Val-Thr-Gln-Tyr-Gln-Gln-Val-Leu-
Ala-Thr-His-Gln-Glu-Ala-Ile-Glu-Lys-Val-Thr655-Glu-Ala-
Leu-Lys-Ile-Asn-Asn-Leu-Arg-Leu-Val-Thr-Leu-Glu-His-Gln-
Val-Leu-Val-Ile-Gly-Leu-Lys-Val-Glu-Ala-Met-Glu-Lys-Phe-
Leu-Tyr-Thr-Ala-Phe-Ala-Met-Gln-Glu-Leu-Gly-Cys*-Asn-Gln-
Asn-Gln-Phe-Phe-Cys*-Lys-Val-Pro-Ser-Ala-Lys-Trp-Glu-Arg-

Tyr-Asn715-Met-Thr-Ile-Asn-Gln-Thr-Ile-Trp-Asn-His-Gly-
Asn-Ile-Thr-Leu-Gly-Glu-Trp-Tyr-Asn-Gln-Thr-Lys-Asp-Leu-
Gln-Gln-Arg-Phe-Tyr-Glu-Ile-Ile-Met-Asp-Ile-Glu-Gln-Asn-
Asn-Val-Gln-Gly-Lys-Lys-Gly-Leu-Gln-Gln-Leu-Gln-Glu-Trp-
Glu-Asp-Trp-Val-Gly-Trp-Ile775-Gly-Asn-Ile-Pro-Gln-Tyr-
Leu-Lys-Gly-Leu-Leu-Gly-Gly-Ile-Leu-Gly-Ile-Gly-Leu-Gly-
Met-Leu-Leu-Leu-Ile-Leu-Cys*-Leu-Pro-Thr-Leu-Val-Asp-
Cys*-Ile-Arg-Asn-Cys*-Ile-His-Lys-Ile-Leu-Gly-Tyr-Thr-
Val-Ile-Ala-Met-Pro-Glu-Val-Glu-Glu-Glu-Glu-Ile-Gln-
Pro835-Gln-Met-Glu-Leu-Arg-Arg-Asn-Gly-Arg-Gln-Cys*-Gly-
Met-Ser-Glu-Lys-Glu-Glu-Glu854

(II)

On entend par épitope, au sens de la présente invention, les épitopes communs à toutes les souches de VIF (zones conservées), reconnus par les anticorps dirigés contre toutes les souches de VIF (reconnaissance de groupe) ; de plus, tous ces fragments induisent une production d'anticorps et/ou une réponse cellulaire.

Ces fragments ont l'avantage de présenter une sélectivité et/ou une spécificité importante vis-à-vis du VIF, tout en étant de petite taille (prix de revient nettement diminué).

Ces peptides sont codés par une séquence ou un fragment de séquence nucléotidique telle que définie dans la séquence de formule I ci-dessus.

Ces peptides TM3 permettent :
- d'un point de vue diagnostic :
   en tant que fragments universels, de reconnaître les anticorps produits par un VIF, s'il est présent, quelle que soit la souche.
- d'un point de vue protecteur (vaccin en particulier) :
   les fragments TM3 induisent plus Spécifiquement la production d'anticorps, et éventuellement une réponse cellulaire ; en conséquence le pool de peptides conforme à l'invention assure soit une protection spécifique vis-à-vis de la souche d'origine, soit une protection plus large (plusieurs souches de VIF).

Au sens de la présente invention le terme fragment peptidique comprend tous les fragments incluant un segment de peptide conforme à l'invention ainsi que les peptides homologues ; de manière générale, on entend par peptide homologue, les fragments peptidiques dont la position et la fonction sont équivalents, au sein du VIF (même localisation que celle définie ci-dessus, sur le génome du VIF) ; en ce qui concerne plus particulièrement les fragments TM3, on entend également par peptides homologues, les peptides qui ne perdent pas les propriétés de reconnaissance des anticorps dirigés contre le peptide de référence.

L'ensemble desdits peptides peut avantageusement être obtenus soit par clonage, soit par synthèse, notamment par synthèse de Merrifield.

La présente invention a également pour objet un vecteur recombinant de clonage et/ou d'expression, caractérisé en ce qu'il comprend une séquence nucléotidique codant pour l'un des peptides TM3 définis ci-dessus.

On entend, au sens de la présente invention, par vecteur recombinant, aussi bien un plasmide, un cosmide, qu'un phage.

Selon un mode de réalisation avantageux dudit vecteur, il est constitué par un plasmide comprenant une origine de réplication, au moins un marqueur de sélection et une séquence nucléotidique codant pour l'un des peptides TM3 définis ci-dessus, lequel vecteur est un vecteur de clonage.

Selon une disposition avantageuse de ce mode de réalisation, ledit vecteur de clonage est constitué par un plasmide capable de se répliquer dans *E*.*coli*, dans lequel est insérée une séquence nucléotidique codant pour l'un des peptides TM3 définis ci-dessus.

Dans le cas où l'on introduit la séquence de formule I dans un vecteur Bluescript® SK⁺, ledit vecteur est dénommé pKSe1.

Ledit vecteur a été déposé auprès de la Collection Nationale de Microorganismes tenue par l'Institut Pasteur, le 12 juin 1992 sous le n° I-1220.

Lorsque ladite séquence est introduite dans un vecteur Bluescript® KS⁺, ledit vecteur est dénommé pKSe.

Selon un autre mode de réalisation dudit vecteur, il est constitué par un vecteur recombinant comprenant une origine de réplication dans un microorganisme hôte approprié, notamment une bactérie ou une cellule eucaryote, au moins un gène dont l'expression permet la sélection soit des bactéries, soit des cellules eucaryotes ayant reçu ledit vecteur, un promoteur permettant l'expression des gènes dans lesdites bactéries ou cellules eucaryotes, et dans lequel est insérée une séquence nucléotidique codant pour l'un des peptides TM3 définis ci-dessus, lequel vecteur est un vecteur d'expression d'un peptide TM3 tel que défini ci-dessus.

Avantageusement, ledit vecteur d'expression est constitué par un phage λgt11 dans lequel est inséré, en phase au niveau de son site, EcoRI, des fragments de digestion à la DNAse du plasmide pKSe1.

La présente invention a également pour objet une composition à pouvoir immunogène et/ou protecteur, caractérisée en ce qu'elle comprend au moins un peptide TM3 tel que défini ci-dessus, éventuellement associé à au moins une autre substance immunogène ou immunostimulante et/ou un véhicule pharmaceutiquement acceptable.

La présente invention, a, de plus, pour objet un procédé de détection d'anticorps anti-VIF, caractérisé en ce qu'il consiste à détecter les anticorps anti-VIF éventuellement présents dans un échantillon biologique à l'aide d'un peptide TM3 tel que défini ci-dessus, éventuellement fixé sur un support solide approprié, en mettant en présence ledit échantillon biologique avec ledit/lesdits peptides, auxquels se lient les anticorps anti-VIF si de tels anticorps sont présents dans l'échantillon à analyser, la lecture du résultat étant révélée par un moyen approprié, notamment EIA, RIA, fluorescence.

Ce procédé permet notamment de vérifier la séroconversion des animaux vaccinés ou de procéder à des enquêtes sérologiques à visée épidémiologique.

A l'aide d'un peptide TM3, ledit procédé permet de déterminer une infection à VIF, quelle que soit la souche (réactif universel tel que précisé ci-dessus).

La présente invention a, en outre, pour objet un kit, prêt à l'emploi, pour la mise en oeuvre du procédé de détection d'anticorps anti-VIF, caractérisé en ce qu'il comprend, outre des quantités utiles de tampons appropriés pour la mise en oeuvre de ladite détection, des doses appropriées d'au moins un peptide TM3 tel que défini ci-dessus.

La présente invention a également pour objet un réactif de diagnostic de l'immunodéficience féline, caractérisé en ce qu'il est choisi dans le groupe constitué par l'un des peptides TM3 tels que définis ci-dessus, et en ce qu'il constitue un réactif de diagnostic universel de souche de VIF.

### EXEMPLE 1 : Séquençage du gène env du virus de l'immuno-déficience féline Wo.

### a) Echantillons d'ADN :

Des cellules mononucléaires de sang périphérique (PBMC), obtenues à partir de chats infectés naturellement par l'isolat français Wo, présentant un syndrome d'immunodéficience acquise féline, sont co-cultivées avec des cellules mononucléaires de sang périphérique, obtenues à partir de chats dits contrôle négatif (chats SPF), en présence d'interleukine-2 humaine recombinante (Genzyme, Boston, USA) et de concanavaline A (Sigma) (MORAILLON A. et al., 1992, Vet. Mic., 31, 41-45, *In vitro properties and experimental pathogenic effect of three feline immunodeficiency viruses isolated from cats with terminal diseases*).

L'activité réverse transcriptase (RT) dépendante de Mg⁺⁺ est plus particulièrement surveillée. Après 15 jours, l'ADN cellulaire total est extrait au phénol (SAMBROOK et al., 1989, Molecular cloning, a laboratory manual), à partir de cultures RT positives et utilisé comme matériel de départ pour l'amplification des gènes du VIF. Ceci permet d'éviter les passages multiples *in vitro* et l'adaptation à des lignées cellulaires CD4 négatives.

### b) PCR :

Les gènes env et *gag* de la souche Wo du virus de l'immunodéficience féline sont respectivement amplifiés en trois et deux fragments chevauchants, en utilisant des amorces synthétiques correspondant à des régions conservées dans les séquences de VIF, isolat *Petaluma* (TALBOTT et al, 1989) et isolat PPR (PHILIPPS et al., 1990).

Les séquences des amorces et les positions (conformément à la numérotation utilisée pour le VIF 34TF10), sont comme suit :
* *env* :
   - pour le premier fragment env, dénommé E₁, on utilise comme amorce 5', le fragment 5'-GCAACAATAAGAATGG-CAGAAGC-3' (6251-6274) et comme amorce 3', le fragment 5'-GTTTAGGGTGACTAGTTAATATGTAAACC-3' (7236-7263) ;
   - pour le second fragment *env*, dénommé E₂, on utilise comme amorce 5', le fragment 5'-CAAATCCCACTGAT-CAATTATACATTTGG-3' (7236-7263), et comme amorce 3', le fragment 5'-CGTAGTTCATGATCATTATCCTAATTTTC-3' (8269-8298) ;
   - en ce qui concerne le fragment dénommé E₃, on utilise comme amorce 5', le fragment 5'-GCATCAAGTAC-TAGTAATAGGATTAAAAG-3' (8269-8298), et comme amorce 3', le fragment 5'-CTTTTTCTCCTCCTTACTACTTC-3' (8809-8833) ;
* *gag* :
   - pour le premier fragment *gag*, dénommé G₁, on utilise comme amorce 5', le fragment 5'-GGAGAGATTCTACAG-CAACATGGGG-3' (608-632) et comme amorce 3', le fragment 5'-GCCAATTTTCCTAATGCCTCGAGATACCATGC-3' (1398-1429) ;
   - pour le second fragment *gag*, dénommé G₂, on utilise comme amorce 5', le fragment 5'-GCATGGTATCTCGAGG-CATTAGGAAAATTGGC63' (1398-1429) et comme amorce 3', le fragment 5'-AATTTACAAATCCAATAGTTTCTCCTCC-3' (1954-2123).

   Les oligonucléotides correspondant à l'amorce 5' du fragment E₂ contiennent un site de restriction BclI et l'oligonucléotide correspondant à l'amorce 3' du fragment E₂ contient un site SpeI, qui permet la reconstruction d'un clone env complet.
   Cette PCR comprend 38 cycles, incluant chacun une dénaturation à 94°C pendant une minute, une hybridation à 55°C pendant une minute et une élongation à 72°C pendant une minute, avec 1 µg d'ADN génomique total, 150 pmol de chaque amorce et 20 nmol de chaque désoxynucléotide triphosphate dans un tampon comprenant du Tris 10 mM, du KCl 50 mM et du MgCl₂ 2 mM.

### c) Clonage et séquençage :

Un vecteur M13mp8 est digéré par l'enzyme SmAI, de manière à pouvoir y insérer les fragments PCR obtenus ci-dessus [par exemple, pour le gène *env* fragments de la séquence de formule I, E₁ (nucléotides 1-977), E₂ (978-2016) et E₃ (2017-2562)]. Le vecteur recombinant obtenu est utilisé pour transformer des *E. coli* JM101 compétents.

Les clones recombinants sont détectés par une hybridation *in situ* avec les fragments PCR correspondants, marqués au phosphore 32.

Le séquençage de l'ADN simple brin à partir de phages recombinants est réalisé par la méthode enzymatique par les didésoxynucléotides, avec un kit de séquençage séquenase® 2.0 DNA, utilisant, comme amorces, des oligonucléotides complémentaires au vecteur (17 mers, amorce universelle) ou l'insert VIF.

Pour reconstituer le gène env complet, les trois fragments (E₁, E₂ et E₃) sont préparés à partir du vecteur recombinant M13mp8 tel que défini ci-dessus, en utilisant les sites de restriction présents dans la séquence de liaison multisites du M13mp8 ou dans les amorces d'amplification : E₁, HindIII-BclI ; E₂, BclI-SpeI ; E₃, SpeI-EcoRI. Les trois fragments sont ligatures dans un vecteur KS⁺ Bluescript® (Stratagène), digéré par les enzymes HindIII et EcoRI, en aval du promoteur T3. La construction obtenue est dénommée pKSe.

Lesdits fragments peuvent également être ligaturés dans un vecteur SK⁺ Bluescript® (Stratagène), digéré par les enzymes HindIII et EcoRI, en aval du promoteur T7. Cette dernière construction, dénommée pKSe1, permet la transcription du gène env dans des cellules de mammifère exprimant le gène de l'ARN polymérase du phage T7, alors que la construction pKSe permet la transcription du gène env dans des cellules de mammifère exprimant le gène de l'ARN polymérase du phage T3.

### d) Analyse des séquences d'aminoacides correspondantes :

L'analyse des séquences est réalisée sur ordinateur avec le programme "Salsa".

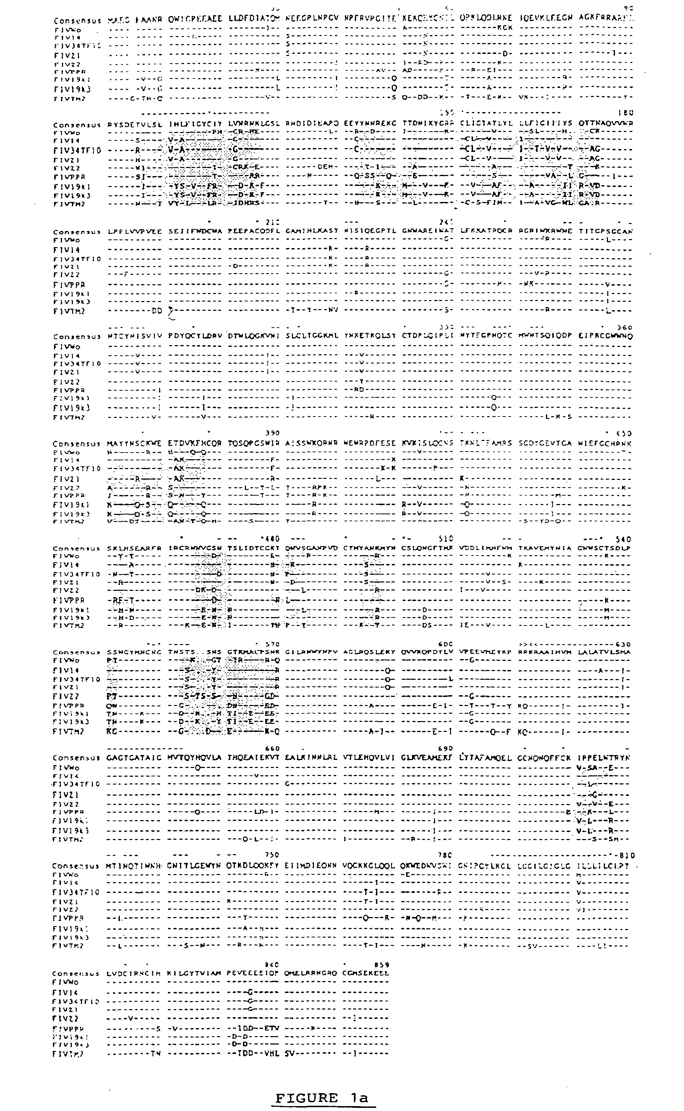

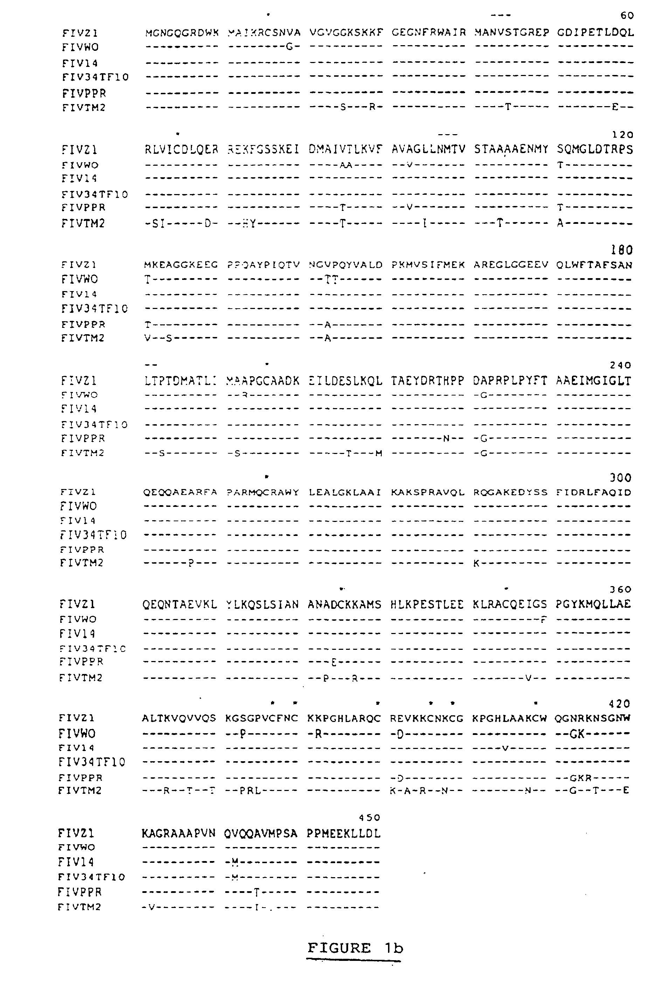

Les multiples alignements comparant la séquence VIF Wo avec d'autres séquences de VIF et notamment VIF34TF10 (USA) (TALBOTT et al., 1989), VIF 14 (USA) (OLMSTED et al., 1989), VIF PPR (USA) (PHILLIPS et al., 1990), VIF TM2/GVEPX (JAPON) (KIYOMASU et al., 1991 ; MIYAZAWA et al.), VIF Z1 et VIF Z2 (SUISSE) (MORIKAWA et al., 1991), VIF 19K1 et VIF 1.9K2 (Pays Bas) (SIEBELINK K.M.J. et al., J. Virol., 1992, 66, 1091-1097) obtenus à partir de la banque de gènes "GenBank", sont illustrés à la figure 1a et 1b, dans lesquelles il apparaît que les variations sont, pour la plupart, situées sur le gène env et sont, en référence auxdites séquences en aminoacides :
- séquence FIV Wo/séquence FIVZ2 : 8,9 %
- séquence FIV Wo/séquence isolat *Petaluma* : 10,2-10,9 %
- séquence FIV Wo/ séquence FIV19 : 10,8 %
- séquence FIV Wo/séquence FIVZ1 : 11 %
- séquence FIV Wo/séquence FIVPPR : 13,6 %
- séquence FIV Wo/séquence FIVGVEPX : 19 %.

Les séquences variables conformes à l'invention sélectionnées (V₁ à V₉, en référence à leurs séquences en aminoacides) sont récapitulées dans le Tableau ci-après ; de manière avantageuse, la sélection de ces fragments permet de définir, par déduction, des zones conservées chez l'ensemble des VIF, et permet de bien mettre en valeur les zones variables et les zones conservées ; ce qui permet de définir des réactifs universels de détection du VIF, des réactifs sélectifs et de faire bénéficier les animaux de la meilleure protection possible :

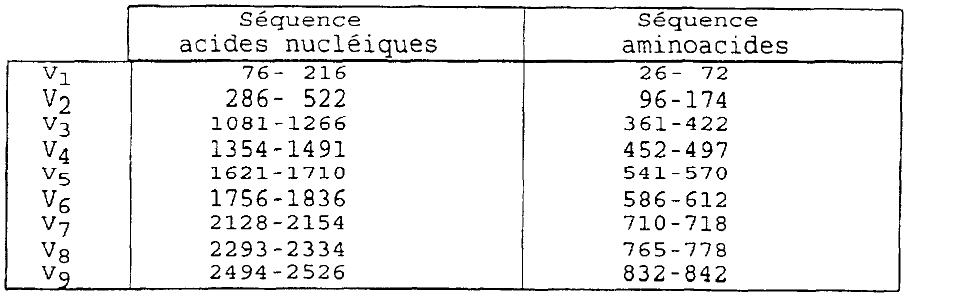

### e) Transcription et traduction in vitro :

Pour la synthèse *in vitro* de l'ARN, les deux constructions pKSe et pKSe1 sont utilisées, la transcription étant effectuée dans le premier cas, par la polymérase T3 et dans le deuxième cas, par la polymérase T7, comme précisé ci-dessus.

1 µg d'ADN des plasmides pKSe1 et pKSe est linéarisé, en utilisant le site de restriction EcoRI, présent dans la séquence de liaison multisites du vecteur, en aval du gène inséré. Le plasmide linéarisé et traité à la protéinase K est transcrit, en utilisant l'ARN polymérase T3 pour pKSe et l'ARN polymérase T7 pour pKSe1, conformément au protocole fourni par le fabricant (Stratagène). Après traitement à la DNase et extraction au phénol, le produit de transcription est précipité à l'éthanol, en présence de 5 µg d'ARNt d'*E. coli*, remis en suspension dans 15 µl d'eau traitée au DEPC et stocké à -70°C. La taille des transcrits est évaluée par électrophorèse sur gel d'agarose à 1 % dans un tampon phosphate 10 mM, pH 7, après une dénaturation au glyoxal/diméthylsulfoxide.

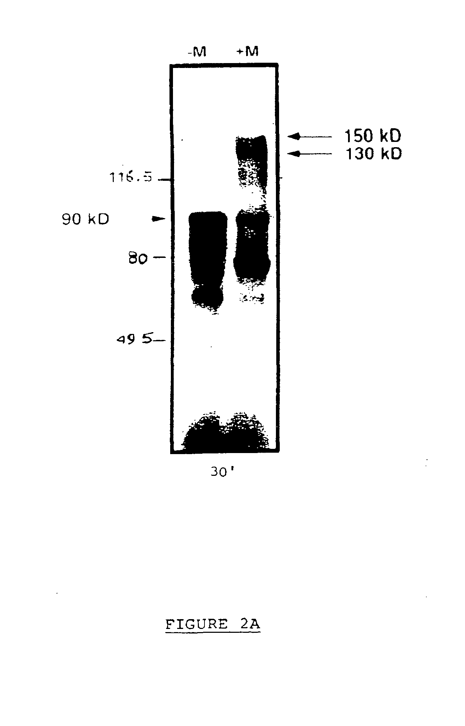

Environ 10 ng de l'ARN transcrit *in vitro* est traduit pendant 30 minutes à 30°C, dans un volume final de 12,5 µg contenant 6 µg de lysat de réticulocytes de lapin (RRL) (Promega), 0,25 µl d'un mélange d'aminoacides dépourvus de méthionine (Promega) et 1,75 µl de méthionine marquée au soufre 35 en l'absence (-M) ou en présence (+M) de membranes microsomales (1 ou 2 µl), pour permettre la glycosylation des protéines (figure 2A). Les 1 ou 2 µl de membranes microsomales pancréatiques de chien (Promega) sont ajoutés par 12,5 µl de mélange de réaction. Pour les essais de cinétique, 3 µl d'aliquots sont prélevés à des moments différents (15, 30, 60 et 90 minutes, 4 heures). Les échantillons sont stockés à -70°C.

Les études d'inhibition en présence de castanospermine, sont réalisées en incubant le mélange de traduction contenant les membranes microsomales (2 µl pour 12,5 µl de mélange) avec de la castanospermine 5 mM (concentration finale) (Sigma), préparée extemporanément dans de l'eau traitée au DEPC, à 30°C pendant 15 minutes, avant l'addition d'ARN.

Les produits de traduction sont dénaturés par ébullition dans un tampon de Laemmli et évalués par électrophorèse (SDS-PAGE, gradient 5-10 %). Des marqueurs du poids moléculaire sont également utilisés (BIORAD H.M.W. ou marqueurs Rainbow). Après l'électrophorèse, les gels sont fixés, traités et autoradiographiés sur un film Kodak XAR-5.

Dans ces conditions, l'ARN transcrit *in vitro* à partir du plasmide pKSe (contenant le gène env de VIF Wo complet) migre, dans un gel d'agarose dans des conditions dénaturantes, en une seule bande d'approximativement 2,5 kb, correspondant à la dimension attendue. Après traduction de ce transcrit dans un lysat de réticulocytes de lapin (RRL), le produit obtenu en plus grande quantité, a un poids moléculaire apparent de 90 kDa (figure 2A), compatible avec le poids moléculaire calculé du polypeptide codé, déduit de la séquence (98,079 kDa).

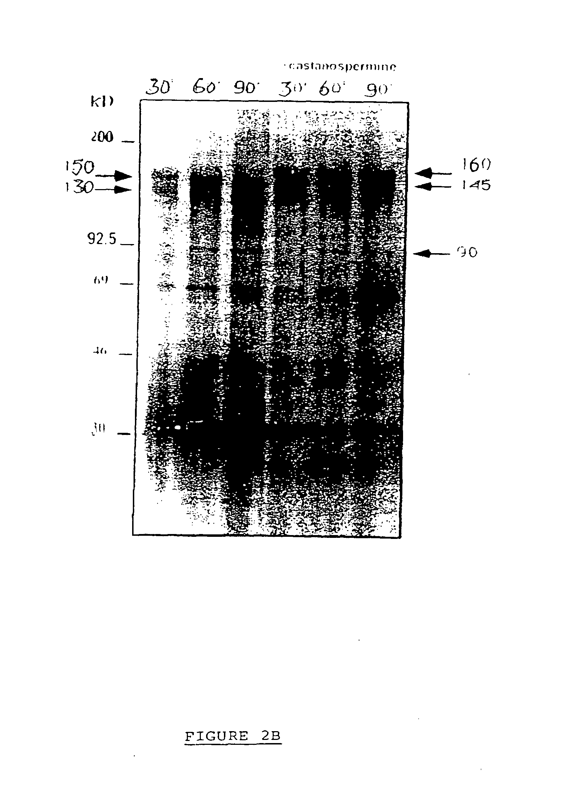

La traduction de l'ARN en présence de microsomes pancréatiques de chien, qui permet une N-glycosylation de la chaîne polypeptidique obtenue, fournit deux produits de poids moléculaires élevés, respectivement de 150 et 130 kDa (figure 2A). Pour analyser la relation entre les deux produits glycosylés obtenus, on réalise des expérimentations étalées dans le temps ; on réalise également, comme précisé ci-dessus, la traduction en présence de castanospermine, un inhibiteur de l'α-glucosidase I du réticulum endoplasmique rugueux, de manière à déterminer si le produit de poids moléculaire le plus bas pourrait dériver de la glycoprotéine de 150 kDa par coupure des sucres pendant la glycosylation. La cinétique de la maturation de l'enveloppe protéinique montre l'apparition, au bout de 15 minutes, des deux produits glycosylés, qui augmente quantitativement jusqu'à 90 minutes d'incubation (figure 2B). En présence de castanospermine, les deux molécules migrent à un poids moléculaire plus élevé, respectivement de 160 et 145 kDa, et restent non modifiées pendant les expérimentations étalées dans le temps (figure 2B).

Ces expériences montrent qu'en l'absence de membranes microsomales qui permettent la glycosylation, on observe un produit de poids moléculaire apparent de 90 kDa, correspondant ainsi au précurseur d'enveloppe non glycosylé. Après addition de membranes microsomales, on observe deux espèces glycosylées de poids moléculaires respectifs 150 kDa et 130 kDa, confirmant la glycosylation importante de ce peptide. La séquence env de Wo contient 21 sites N-glycosylation potentiels, qui sont probablement utilisés complètement comme le suggère les poids moléculaires des produits de traduction, en présence de membranes microsomales.

### f) Immunoprécipitation :

Des anti-sérums de VIF sont obtenus à partir de deux chats SPF (chats dépourvus de pathogènes spécifiques), inoculés avec des isolats Wo ou *Petaluma* et à partir également de deux chats infectés naturellement, mais séronégatifs vis-à-vis du virus de la leucémie féline. Des sérums de contrôle sont obtenus à partir des deux chats SPF. Pour l'étape de la préabsorption, un stock de sérums de chats SPF est utilisé. Des anticorps polyclonaux de lapin dirigés contre des peptides synthétiques tels que décrits dans la Demande de Brevet français n° 2 669 338 sont également utilisés, à des concentrations de 20 µg/ml. Le premier de ces peptides (P100), d'une longueur de 21 aminoacides, est situé à l'extrémité C terminale de la glycoprotéine de surface du VIF et inclut le site de Coupure entre la glycoprotéine de surface (SU) et la glycoprotéines transmembranaire (TM) ; le deuxième peptide (P102), d'une longueur de 25 aminoacides, correspond à l'extrémité C terminale de la glycoprotéine TM du VIF. Des sérums de lapin préimmunisés sont utilisés pour l'étape de préabsorption et comme contrôle négatif dans les essais d'immunoprécipitation.

Des aliquots de produits de traduction in vitro (approximativement 100 000 cpm, déterminés après précipitation à l'acide trichloroacétique) sont dilués dans 200 µl d'un tampon RIPA (Triton X-100 1 %, sodium désoxycholate 0,5 %, NaCl 150 mM, EDTA 2 mM dans Tris-HCl pH 7,5, 50 mM). Les réactions sont réalisées à 4°C. Les échantillons sont préabsorbés pendant 30 minutes avec 4 µl d'un pool de sérums de chats SPF ou avec un sérum de lapin préimmunisé et précipités avec 50 µl de protéine A-Sépharose CL-4B (PrA, Pharmacia). Les surnageants sont alors incubés pendant une nuit avec 4 µl de sérum de chats infectés, de sérum de chats SPF ou d'anticorps polyclonaux de lapin anti-peptide synthétique tel que défini ci-dessus. 50 µl de PrA sont ajoutés et incubés pendant 1 heure. Après 4 lavages dans un tampon RIPA, les culots des immunocomplexes-PrA sont élués par ébullition pendant 5 minutes dans 50 µl de tampon Laemmli. Les échantillons sont analysés sur un gradient SDS-PAGE 5-10 %.

Des cellules FL-4, dérivées des cellules mononucléaires de sang périphériques (PBMC) infectées par une souche *Petaluma* et produisant spontanément le VIF (Yamamoto,1991), sont lavées dans du PBS froid et lysées dans un tampon RIPA. Les lysats cellulaires sont clarifiés par centrifugation à 17000g pendant 30 min.

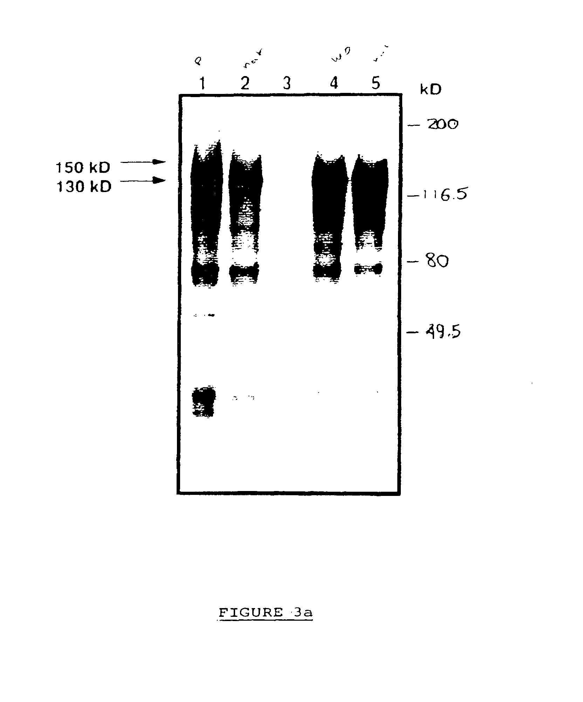

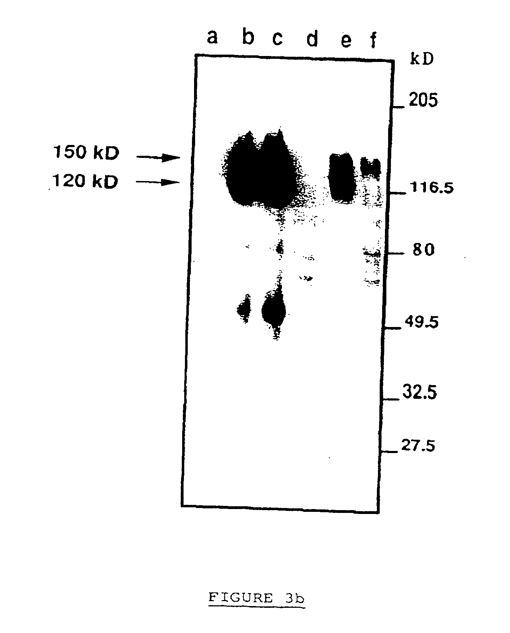

Les produits de traduction env mature obtenus in vitro, sont soumis à une immunoprécipitation (figure 3) avec les différents sérums d'animaux précités (sérums SPF, inoculés expérimentalement avec des isolats Wo et Petaluma (bandes 1 et 4), sérums de chats infectés naturellement (bandes 2 et 5), sérums de chats SPF non infectés, comme contrôle négatif (bande 3)). Cette figure 3 illustre les résultats obtenus ; les deux produits glycosylés (150 kDa et 130 kDa) sont immunoprécipités par les sérums de chats infectés, le produit de 130 kDa étant le produit majeur. La bande de 90 kDa représente probablement un matériel non glycosylé résiduel. Le produit de poids moléculaire le plus bas, peut être considéré comme un produit de transcription ou de traduction partielle. Aucune bande n'est détectée dans les sérums de chats SPF et non infectés.

La figure 3b illustre, en comparaison, l'immunoprécipitation de protéines VIF marquées métaboliquement, obtenues à partir de cellules FL-4. Les bandes a, b et c représentent le résultat obtenu avec des sérums de chats SPF non infectés, des sérums de chats infectés avec une souche *Petaluma* et des sérums de chats infectés par le VIF Wo. Les bandes d, e et f correspondent à un sérum de lapin préimmune, des sérums de lapin polyclonaux dirigés contre le peptide C-terminal de la protéine SU (de l'aminoacide 6 à la portion TM(P100)) et des sérums de lapin polyclonaux dirigés contre un peptide C-terminal de la protéine TM (P102). Les bandes b et c permettent la détection de deux protéines (150 kDa et 115-125 kDa), qui correspondent à la protéine précurseur d'enveloppe et à la glycoprotéine SU, respectivement, comme le confirme l'immunoprécipitation avec les deux anticorps de lapin dirigés contre les peptides incluant le site de clivage SU-TM ou correspondant à la partie C-terminale du précurseur *env* (bandes e et f).

### EXEMPLE 2 : Test de dépistage d'une infection à VIF.

On hybride in situ les fragments d'acide nucléique isolés à partir d'un échantillon à tester et transféré sur un filtre de nitrocellulose, avec un fragment d'acide nucléique conforme à l'invention (sonde marquée avec du dATP ³²P), à 58°C pendant une nuit, après blocage des liaisons non spécifiques, avec une solution de Denhardt.

Un tel procédé, mis en oeuvre avec un pool de fragments dits variables (V₁-V₉) permet une différenciation spécifique de souche de VIF.

### EXEMPLE 3 : Peptides correspondant à des épitopes ± conservés (TM3) conformes à l'invention.

### a) Construction d'une banque d'expression des peptides conformes à l'invention :

Le clonage du gène *env* du VIF Wo dans un vecteur Bluescript® KS⁺ fournissant la construction pKSe ou dans un vecteur Bluescript® SK⁺ fournissant la construction pKSe1 ont été décrits à l'exemple 1.

Des fragments chevauchants du plasmide pKSe sont générés par digestion à la DNase I en présence d'ions manganèse. Les fragments d'ADN obtenus, après un temps optimal d'incubation avec la DNase (temps donnant des fragments de longueur d'environ 200 pb) sont traités avec le fragment de Klenow de la DNA polymérase d'*E*. *coli*, de manière à obtenir des extrémités à bouts francs, pour une ligature efficace avec la séquence de liaison. Le marquage au phosphore 32 des fragments d'ADN permet le contrôle des étapes ultérieures.

Des séquences de liaison de 10 paires de base (pb), ayant un site EcoRI (Pharmacia), sont utilisées pour la ligature avec les fragments pKSe obtenus. Les produits de la ligature sont alors digérés avec l'enzyme de restriction EcoRI et séparés par électrophorèse dans des gels d'agarose LMP NUSieve 2 %, de manière à sélectionner un ensemble de fragments comprenant entre 100 et 200 pb et à éliminer les séquences de liaison libres.

Les fragments sélectionnés sont extraits au phénol à partir de l'agarose et ligaturés avec un vecteur λgt11 digéré à l'enzyme de restriction EcoRI (Promega). Les produits de la ligature sont encapsidés en utilisant un kit Packagene (Promega) et étalés sur une souche d'*E*. *coli* Y1090. Les phages recombinants sont sélectionnés par détection d'une couleur en présence de 25 µl d'IPTG 1 M et de 25 µl de Xgal 80 mg/ml. L'étalement de la banque λgt11 fournit 10⁵ clones indépendants. L'ADN de phage, obtenu à partir des plaques non colorées, est analysé par PCR en utilisant des amorces λgt11 1218 et 1222, complémentaires du fragment β-galactosidase de la matrice de λgt11 : 8 des 10 clones contiennent des inserts de dimension moyenne d'environ 160 pb. La banque est alors amplifiée pour obtenir un titre de 3 x 10⁹ par ml.

Une hybridation *in situ* de 37 phages avec des sondes d'ADN marquées au phosphore 32, représentant une amplification PCR des 3 régions E₁, E₂ et E₃ du gène *env* Wo (fragments 1-977 ; 978-2016 ; 2017-2562), indiquent que la banque est représentative du gène env entier.

### b) Criblage de la banque :

- La banque *env* λgt11 est criblée en utilisant des sérums de deux chats expérimentalement infectés avec l'isolat VIF Wo. La banque est étalée à une concentration approximative de 3x10⁴ pfu sur *E. coli* Y1090 et les plaques sont incubées à 42°C pendant 3 à 4 heures. Les plaques sont alors recouvertes de filtres de nitrocellulose saturés d'IPTG 10 mM et incubés pendant 3 heures à 37°C. Les filtres sont traités pour un criblage immunologique avec des sérums félins, dénommés CTF 1915 et CTF 1916, dilués au 300ème en utilisant des méthodes standards [procédure au lait dégraissé (MANIATIS)]. Des IgG de chèvre anti-chat (H+L) couplées à la peroxydase (KPL) et diluées au 1000ème, sont utilisées comme deuxième anticorps. Une réaction positive est révélée par du 4-chloro-1-naphtol (Merck) (bruit de fond peu important).
Des fragments d'agar, contenant les particules de phages obtenues à partir de régions correspondant à des signaux positifs sur le filtre, sont prélevés et incubés avec un 1 ml de SM qui comprend :

| | |
|---|---|
| NaCl | 2,9 g |
| MgSO₄ | 2 g |
| Tris 1M pH 7,5 | 2,5 ml |
| Gélatine 2 % | 2,5 ml |
| H₂O | 500 ml |

Les phages sélectionnés sont réensemencés pour un deuxième criblage. Les plaques de phages positives sont prélevées et incubées avec 1 ml de SM. La réactivité des phages sélectionnés est finalement confirmée par une troisième procédure de criblage. Les lysats de phages λ obtenus à partir de phages positifs sont préparés par une méthode d'étalement standard (protocole Promega) et stockés à 4°C en présence de chloroforme 0,3 %. L'analyse subséquente des inserts env est réalisée soit par séquençage direct des amplifications PCR ou par hybridation *in situ* en utilsant des sondes marquées au phosphore 32, comme précisé à l'exemple 1.
- Le criblage immunologique proprement dit de la banque est réalisé avec les deux sérums cités ci-dessus. 170 plaques positives sont détectées au premier criblage, 108 de ces plaques sont isolées après le troisième criblage ; 60 clones sur les 108 sont séquencés et les autres sont analysés par une hybridation *in situ* avec des sondes radioactives correspondant aux régions immunogéniques majeures déjà séquencées, comme spécifié ci-dessus.
Pour l'hybridation *in situ*, l'ADN de bactériophage est transféré sur un filtre de nitrocellulose et hybridé avec des sondes marquées au phosphore 32 à 58°C, pendant une nuit, après le blocage des liaisons non spécifiques avec une solution de Denhardt. Comme sondes, on utilise des produits de l'amplification des clones de VIF 1, 2, 9, 35, 44, 90, 133, 147 et 170 marqués avec du dATP ^{[32]}P, en utilisant la méthode de translation de coupure.

En utilisant cette double stratégie, 85 inserts ont été sélectionnés comme correspondant à 6 régions immunologiques de l'enveloppe comme visible dans les Tableaux I et II ci-après, dans lesquels le Tableau I présente les séquences des peptides antigéniques exprimés par les clones de bactériophages recombinants et le Tableau II précise la distribution des clones positifs, dans les régions immunogènes.

**TABLEAU I**

| Protéine virale | Numéro de clone | Réactivité | Séquence^{(a)}nucléotidique | Séquence^{(b)}peptidique |
|---|---|---|---|---|
| gp120 SU1 (1ère région) ^{(d)} | 96 | + ^{(c)} | 727-873 | 244-291 |
| | 147 | ++ | 735-890 | 246-296 |
| | 14 | ++ | 747-872 | 250-290 |
| | 97 | + | 750-870 | 251-289 |
| | 54 | ++ | 755-872 | 253-290 |
| gp120 SU2 (2ème région) | 93 | ++ | 1093-1274 | 365-424 |
| | 69 | ++ | 1113-1274 | 372-424 |
| | FIV1 | ++ | 1122-1274 | 375-424 |
| | 12 | ++ | 1142-1273 | 382-424 |
| | 133 | ++ | 1160-1274 | 387-424 |
| | 75 | ++ | 1163-1274 | 388-424 |
| gp120 SU3 (3ème région) | 73 | ++ | 1377-1536 | 460-512 |
| | 148 | + | 1386-1506 | 463-502 |
| | 154 | ++ | 1387-1538 | 463-512 |
| | 170 | +/++ | 1398-1478 | 467-492 |
| | 48 | + | 1452-1585 | 485-528 |
| SU4 | 131 | + | 1452-1599 | 485-533 |
| | 136 | + | 1458-1598 | 487-532 |
| | 64 | + | 1460-1597 | 488-532 |
| | 44 | +/++ | 1492-1597 | 498-532 |
| | 100 | +/- | 1502-1637 | 502-545 |
| | 158 | + | 1506-1637 | 503-545 |
| | 130 | + | 1521-1701 | 508-567 |
| gp120 SU5 (4ème région) | 23 | + | 1654-1820 | 552-606 |
| | 103 | + | 1657-1820 | 553-606 |
| | 35 | ++ | 1683-1823 | 562-607 |
| | 91 | ++ | 1700-1822 | 568-607 |
| | 51 | ++ | 1714-1822 | 572-607 |
| gp120 SU/ 41 TM1 (4ème région) | 42 | ++ | 1719-1872 | 574-623 |
| | 124 | + | 1782-1944 | 595-647 |
| | 20 | +/- | 1789-1939 | 597-646 |
| gp41 TM2 (5ème région) | 88 | + | 1961-2134 | 654-711 |
| | 53 | + | 1299-2153 | 667-717 |
| | 163 | + | 2002-2215 | 668-738 |
| | 84 | + | 2011-2176 | 671-725 |
| | 157 | ++ | 2019-2176 | 674-725 |
| | FIV2 | ++ | 2019-2179 | 674-726 |
| | 87 | + | 2020-2167 | 674-722 |
| | 160 | + | 2020-2140 | 674-713 |
| | 108 | ++ | 2039-2209 | 681-736 |
| | 63 | ++ | 2092-2285 | 698-761 |
| | 11 | ++ | 2097-2282 | 700-760 |
| TM3 | 68 | ++ | 2118-2272 | 707-757 |
| | 33 | ++ | 2175-2365 | 726-788 |
| | 9 | ++ | 2226-2366 | 743-788 |
| | 168 | ++ | 2230-2369 | 744-789 |
| gp41 TM4 (6ème région) | 27 | ++ | 2427-2562 | 810-854 |
| | 90 | + | 2475-2562 | 826-854 |

| | | | | |
|---|---|---|---|---|
| ^{(a)} séquences inserts déterminées à partir des clones d'ADN. La numération des nucléotides commence à partir du codon d'initiation ATG. | | | | |
| ^{(b)} les positions des aminoacides sont numérotées à partir de la méthionine N-terminale. | | | | |
| ^{(c)} l'intensité des réactions immunologiques est évaluée de faible (+/-) à forte (++). | | | | |
| ^{(d)} les régions immunogènes de l'enveloppe déterminées à l'aide des séquences chevauchantes des clones réactifs. | | | | |

Il faut noter que la protéine SU (glycoprotéine d'enveloppe externe de surface) présente un poids moléculaire de 100 à 120 et que la protéine TM (glycoprotéine virale d'enveloppe transmembranaire) présente un poids moléculaire compris entre 35 et 45.

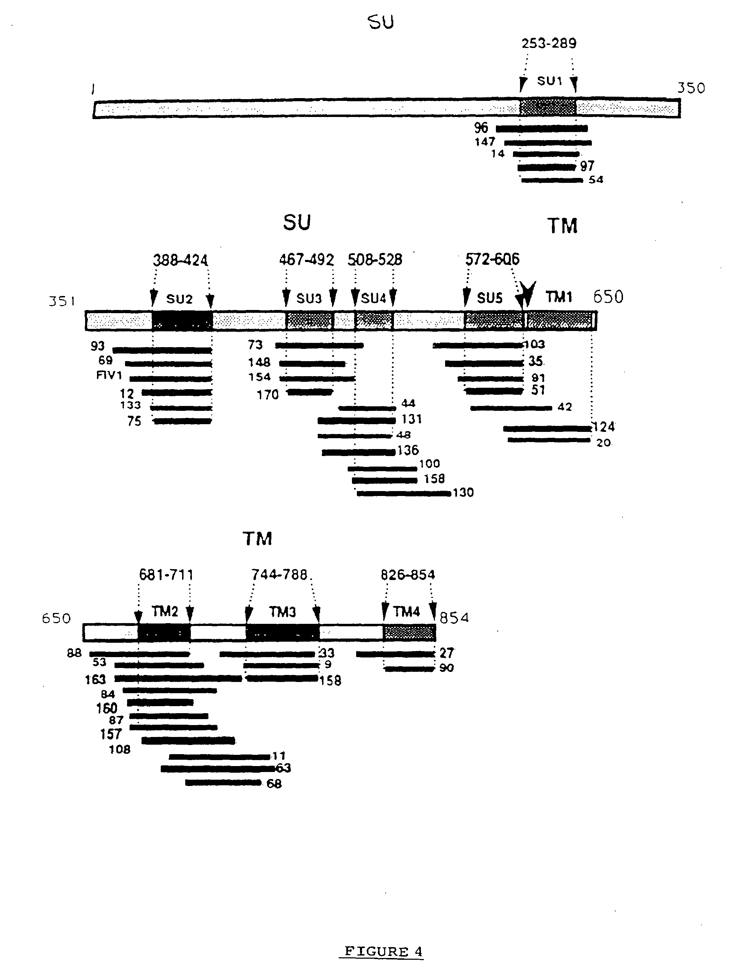

Le Tableau I et la figure 4 précisent les séquences nucléotidiques des différents inserts ainsi que les séquences peptidiques correspondantes.

Les séquences chevauchantes mettent en valeur la présence de 8 domaines immunogènes (épitopes), dont 5 sont situées dans la glycoprotéine extramembranaire (gp) SU (SU1, aminoacides 253-289 ; SU2, aminoacides 388-424 ; SU3, aminoacides 467-492 ; SU4, aminoacides 508-528 et SU5, aminoacides 572-606) et 3 dans la gp transmembranaire TM (TM2, aminoacides 681-711 ; TM3, aminoacides 744-788 et TM4, aminoacides 826-854) (figure 1). La séquence peptidique 597-646, qui est définie par les clones 20 et 124, chevauche le 5ème épitope SU (574-606) et représente, sans doute, un épitope TM NH₂-terminal (TM1). Cette hypothèse est confirmée par le criblage immunologique avec les sérums de chats, qui montre les différentes réactivités entre le clone 51 (épitope SU5) et le clone 124 (épitope TM1).

Cette figure 4 illustre la localisation des régions épitopiques des glycoprotéines (gp) d'enveloppe du VIF, avec SU:gp100 ; TM:gp40. Dans cette figure, les sites de clivages sont représentés par des flèches verticales ; les clones principaux sont représentés par des lignes épaisses et les différents rectangles indiquent les épitopes correspondants aux séquences chevauchantes minimales (rectangle de couleur sombreLe Tableau II, ci-après, illustre le nombre de clones présentant les régions immunogéniques telles que précisées au Tableau I ci-dessus.

**TABLEAU II**

| Régions immunogéniques | Nombre de clones |
|---|---|
| 1ère | 7 |
| 2ème | 11 |
| 3ème | 22 |
| 4ème | 10 |
| 5ème | 31 |
| 6ème | 4 |

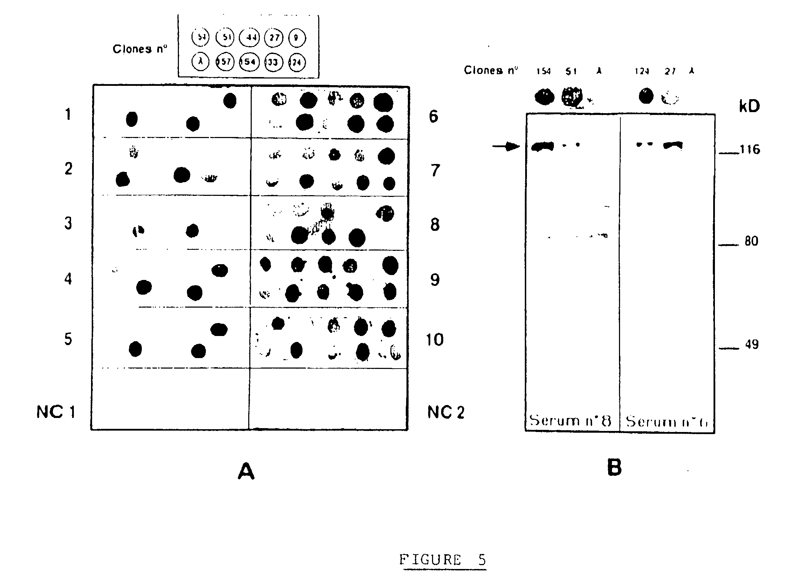

Les phages présentant la même séquence sont comptés une fois et les phages contenant un ou plusieurs inserts ou qui hybrident avec plus d'une sonde sont éliminés.
- Le criblage sérologique des épitopes identifiés est réalisé à partir de sérums de 24 chats infectés expérimentalement avec différents isolats de VIF et à partir de 24 chats naturellement infectés (2 de ceux-ci étant également co-infectés par le virus de la leucémie féline) (Tableau III). Tous les sérums ont été considérés comme réactifs avec les protéines structurales virales lorsqu'ils sont testés en Western blot. 8 sérums de chats SPF sont utilisés comme contrôle. Les clones de bactériophages 54, 133, 154, 44, 51, 124, 157, 9 et 27, du Tableau I, contenant les inserts correspondant aux domaines épitopiques identifiés, ont été également criblés en utilisant un essai dot-phage, (figures 5A et 5B) comme suit :
   Approximativement 100 pfu de chaque phage dans 1 µl de SM sont étalés sur une couche de *E. coli* Y1090 et mis en culture jusqu'à ce que des plaques apparaissent.
   Pour le criblage immunologique, un filtre de nitrocellulose imprégné d'IPTG est placé sur la couche et on réalise les étapes comme décrit précédemment pour le criblage de la banque.
   Les résultats sont illustrés sur le Tableau III ci-après et sur les figures 5.
   A la figure 5A, chaque filtre de nitrocellulose correspond à un test sérique.
   Les tests sont réalisés comme précisés ci-dessus et la révélation de la réaction se fait avec du 4-chloro-1-naphtol jusqu'à ce que le bruit de fond des échantillons contrôle devienne visible (filtre λ : phage non recombinant utilisé comme contrôle négatif).
   Les numéros de clones sont indiqués en haut de la figure (54, 51, 44, 27, 9 et λ, 157, 154, 133, 124).
   Deux tests sont plus particulièrement représentés à cette figure (1-5 et 6-10), NC₁ et NC₂ étant des contrôles négatifs (chats SPF) ; 1, 7 et 10 : chats inoculés par du VIF Villefranche ; 2 et 3 : chats infectés naturellement ; 4 et 9 : chats inoculés avec du VIF ENVNIP ; 5 : chats inoculés par du VIF *Petaluma* ; 6 : chats inoculés par un VIF Me ; 8 : chat inoculé par un VIF Wo. 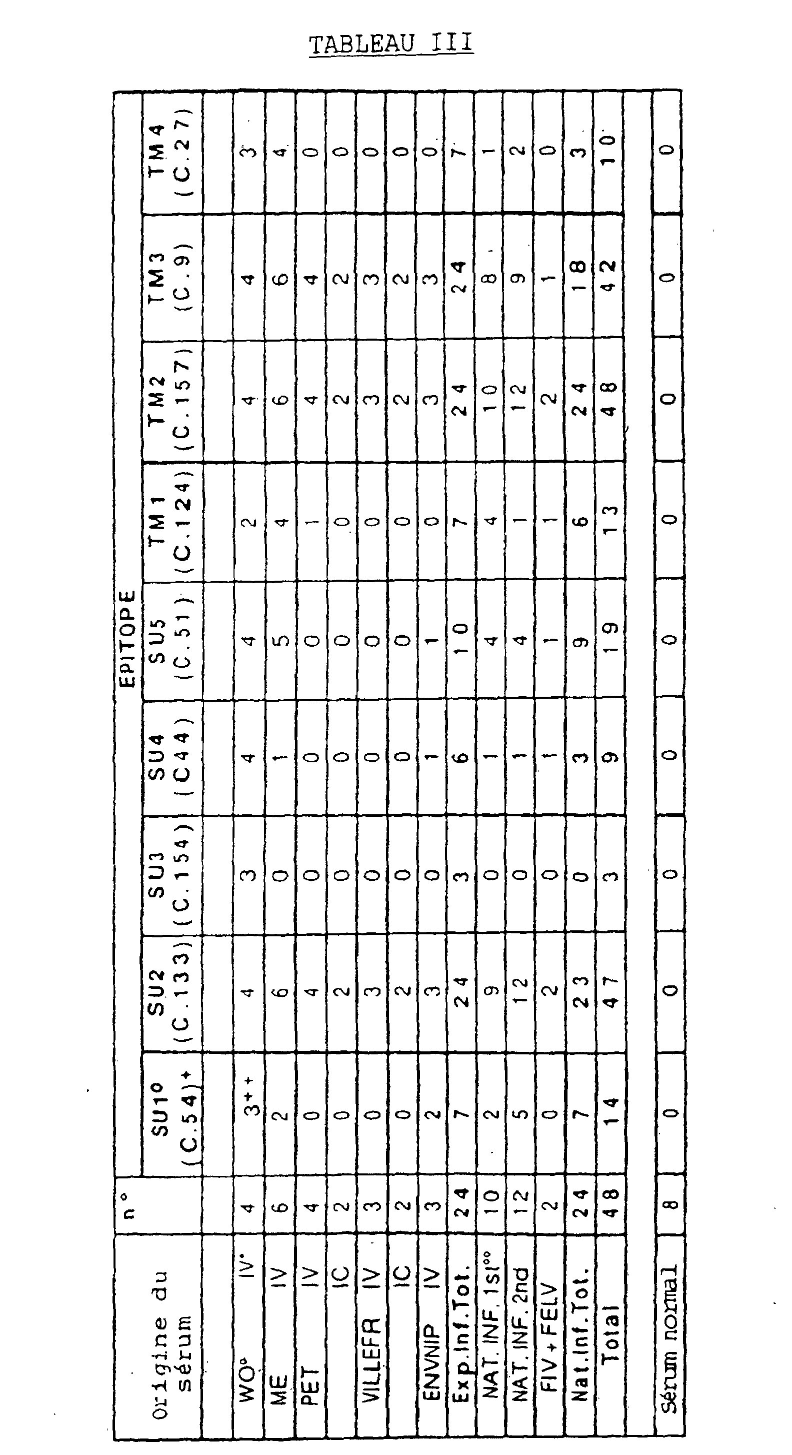
   Le Tableau III, dans lequel PET = *Petaluma* ; Villefr = Villefranche ; NAT. INF. 1st = chats naturellement infectés provenant de l'école vétérinaire de Nanties ; NAT. INF. 2nd = chats naturellement infectés provenant de l'école vétérinaire de Maisons-Alfort ; IV = inoculation intraveineuse ; IC = inoculation intra-cérébrale, montre la réactivité des régions épitopiques avec des sérums de chats SPF et infectés : le clone 133 (épitope SU2) est réactif avec tous les sérums de chats infectés expérimentalement et avec 23/24 sérums de chats naturellement infectés. Le clone 157 (épitope TM2) est reconnu par la totalité des sérums de chats infectés. Pour ces deux clones, l'intensité de réaction est très importante. Le clone 9 (épitope TM3) est réactif avec 100 % des sérums de chats infectés expérimentalement et avec 75 % des sérums de chats infectés naturellement ; le clone 27 (TM4) réagit avec 29 % des sérums de chats infectés expérimentalement (1 Wo et 4 Me) mais seulement avec 12,5 % de chats infectés naturellement. Le clone 44 (SU4) réagit avec 25 % des sérums de chats infectés expérimentalement et avec 12,5 % des chats infectés naturellement.
   Les autres épitopes montrent une réactivité variable sans différence significative entre les infections naturelles et expérimentales : 29 % SU1, 39,5 % SU5 et 27 % TM1. SU3, qui est détecté par les deux sérums de chats infectés Wo utilisés pour le criblage de la banque, réagit seulement avec un seul des deux autres sérums testés (des chats infectés par Wo) et ne réagit du tout avec les sérums d'autre origine. Aucune réactivité n'est détectée avec les sérums normaux.
- La réactivité en dot-phage des sérums de chats infectés avec les peptides exprimés par les clones sus-dits est confirmée par le Western blot (figure 5B) réalisé avec des lysats d'*E. coli*, contenant les antigènes recombinants exprimés par les phages lysogènes. La figure 5B illustre cette réactivité. Les sérums utilisés sont les mêmes que ceux de la figure 5A n° 6 et n° 8. Les numéros de clones sont indiqués en haut de la figure.

De manière plus précise, les clones recombinant λgt11 54, 133, 73, 154, 44, 51, 124, 157, 9 et 27 sont exprimés en tant que lysogènes dans *E. coli* Y1089, comme décrit par HUYNH et al. (1985). Du lysat brut, contenant les protéines de fusion, est préparé à partir de culture lysogénique induite par IPTG en faisant des cycles congélation-décongélation et sonication du culot cellulaire, remis en suspension dans 200 µl d'un tampon TEP (Tris-HCl 100 mM, pH 7,4, EDTA 10 mM, PMSF 1 mM).

Les extraits sont portés à ébullition dans un tampon Laemmli et remis en suspension dans des gels de SDS polyacrylamide 5-10 % (40 µg/puits) en utilisant un appareil Mini PII (BIO-RAD). Après le transfert des protéines sur un filtre de nitrocellulose et le blocage des sites non spécifiques par incubation avec du lait délipidé à 3 % dans du Tris 10 mM, NaCl 150 mM, Tween 20 0,1 % (tampon TNT), des bandes de nitrocellulose sont incubées avec le sérum de chats (dilué au 300ème) pendant 2 heures à 37°C. Les anticorps liants sont révélés par incubation avec des IgG de chèvre couplés à de la peroxydase anti-chat (dilution au 1 000ème) suivie par une révélation avec du 4-chloro-1-naphtol (Merck). Les contrôles sont constitués par un lysat de *E. coli* Y1089 infecté par du λgt11 et par un pool de sérums normaux.

### c) Analyse comparative des séquences :

L'analyse des séquences est réalisée sur ordinateur avec le programme "Salsa" (voir exemple 1, f) pour les pourcentages comparatifs entre différentes souches et figures 1a et 1b).

Dans la glycoprotéine SU de *env*, l'hypervariabilité concerne successivement les segments 361-377, qui s'étend de part et d'autre d'une cystéine conservée en position 367, 4 fragments courts 452-455, 466-471, 479-485 et 494-496, le dipeptide 541-542 et le segment 553-570, qui comprend une cystéine conservée en position 566. Deux zones hypervariables ont également été localisées dans la protéine TM : résidus 710-718 et 832-842. Des régions variables se trouvent dans le domaine N-terminal *rev-like*, dans la portion C-terminale de la SU et dans la portion C-terminale du domaine transmembranaire hydrophobe de la TM (entre les positions 220 et 286) et présentent une variabilité en dessous du seuil de 10 %.

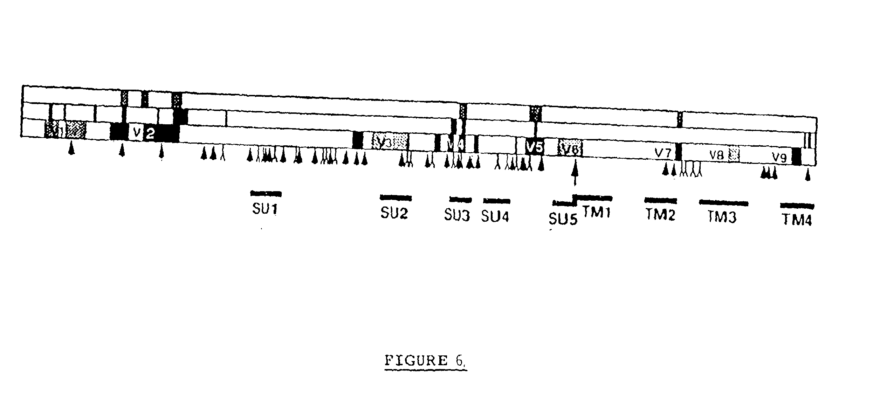

De manière plus précise, de tels peptides comprennent avantageusement, les 9 régions variables telles que définies ci-dessus (régions hypervariables+régions variables adjacentes), à savoir : V1 (résidus 26-72), V2 (résidus 96-174, 23 % de divergence), V3 (résidus 361-422, comprenant la région hypervariable 361-391 et la région variable 392-422, 12 % de divergence), V4 (résidus 452-497, 13 % de divergence), V5 (résidus 541-570, 30 % de divergence), V6 (résidus 586-612, 10 % de divergence), V7 (résidus 710-718, 26 % de divergence), V8 (résidus 765-778, 12 % de divergence) et V9 (résidus 832-842, 21 % de divergence), comme le montre la figure 6 qui illustre les domaines variables et les domaines conservés : les domaines conservés sont représentés en blanc, les régions variables en gris, et les régions hypervariables en noir.

### EXEMPLE 4 : Test de dépistage d'une infection à VIF.

Des peptides correspondants aux épitopes immunodominants (SU2, TM2 ou TM3) sont absorbés dans les puits d'une plaque ELISA.

Le sérum à tester est incubé à une dilution optimale (prédéterminée lors de la mise au point de l'E-LISA, en utilisant une série de sérums de chats infectés et de chats SPF). La présence d'anticorps anti-VIF est révélée par des anticorps anti-Ig de chat couplés à de la peroxydase de raifort, en présence d'un substrat chromogène.

### 1) Protocole opératoire :

### a) Peptides sélectionnés :

Les peptides sélectionnés sont les peptides P237, P240, P241 et P242 ; ils correspondent respectivement, comme précisé ci-dessus, à :
- P237, fragment inclus dans TM2, correspondant aux positions 697-705 de la séquence de formule II,
- P240, fragment inclus dans SU2, correspondant aux positions 398-408 de la séquence de formule II,
- fragments P241 et P242, inclus dans TM3, et correspondant respectivement aux positions 763-770 et 772-779 de la séquence de formule II.

Ces différents peptides sont testés séparément, vis-à-vis de 17 sérums issus de chats infectés naturellement, vis-à-vis de 16 sérums de chats infectés expérimentalement avec différentes souches de VIF et vis-à-vis de sérums de 11 chats négatifs (chats SPF).

### b) Conditions du test ELISA :

Les peptides sont utilisés à des concentrations comprises entre 1 et 10 µg/ml ; pour effectuer le revêtement des plaques de microtitration avec les peptides, du carbonate de sodium 0,1 M, pH 9,6 PBS ou des microplaques préactivées à la poly-L-lysine sont utilisés.

La saturation des sites libres est réalisée avec des concentrations différentes de lait dégraissé ou de sérum albumine bovine.

Les différents sérums sont dilués de 1:1 à 1:200.

Le test se déroule comme suit : 0,25 µg de peptide dans 50 µg de carbonate de sodium 0,1 M, pH 9,6 sont adsorbés sur les puits d'une plaque de microtitration (DYNATECH IMMULON® 2) pendant une nuit à 4°C. Les puits sont ensuite lavés trois fois avec du PBS. Les sites d'adsorption résiduels sur les microplaques sont saturés par incubation avec 100 µl de PBS contenant du lait (1 %) et du Tween 20 (0,1 %) (Tampon ELISA, EB), pendant 2 heures. 50 µl de sérum de chat dilué sont incubés dans les puits pendant 2 heures à température ambiante.

Après lavage trois fois avec du tampon EB, des immunoglobulines-anti-chat conjuguées à la peroxydase (0,5 µg/ml) (KIRKEGAARD & PERRY LABORATORIES, INC) sont ajoutées pendant une heure à température ambiante. Après 5 lavages au PBS, le conjugué à la peroxydase adsorbé est visualisé avec de l'acide 2,2'-azino-bis (3-éthylbenz-thiazoline-6 sulfonique) (ABTS) (Sigma) 0,2 mg/ml dans de l'acide acétique à 0,6 %, pH 4,7 et à une concentration finale (p/v) de H₂O₂ de 0,1 %.

### 2) Résultats

Les essais sont réalisés en double.

Pour discriminer les réactions positives des négatives, une valeur limite est calculée comme suit : P = m_{NC} + 3 x Sd (absorbance moyenne pour des sérums SPF négatifs contrôles (NC) + 3 déviations standard). Les valeurs d'absorbance supérieures à P sont considérées comme indiquant la présence d'anticorps vis-à-vis desdits peptides.

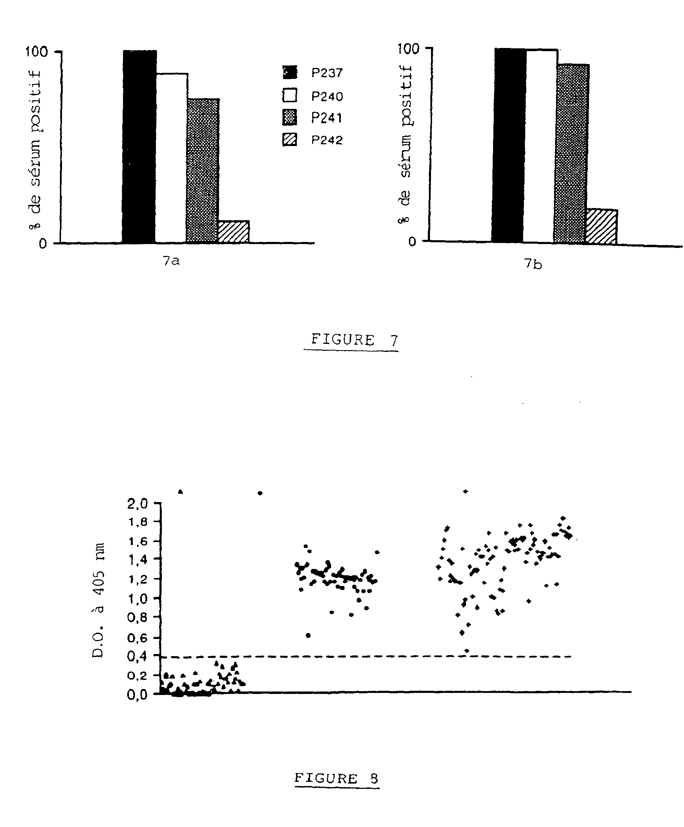

Le test ELISA (figures 7a et 7b, □), utilisant le fragment P240, détecte 100 % des sérums de chats naturellement infectés et 87,5 % des sérums de chats infectés expérimentalement (P = 0,156 (0,084 + 3 x 0,024)).

Le fragment P237 (figures 7a et 7b, ■) est reconnu par 100 % des sérums de chats infectés naturellement et expérimentalement (P = 0,294 (0,138 + 3 x 0,052)).

Le fragment P241 (figures 7a et 7b, 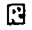 ) est réactif vis-à-vis de 94 % des chats naturellement infectés et vis-à-vis de 75 % des sérums de chats infectés expérimentalement (P = 0,443 (0,209 + 3 x 0,078)).

Le fragment P242 (figures 7a et 7b, 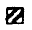 ) est réactif vis-à-vis de 12 % des chats naturellement infectés et vis-à-vis de 17 % des chats expérimentalement infectés (P = 0,359 (0,149 + 3 x 0,070)).

Tous les sérums de chats SPF gardent une valeur de P inférieure à celle de chacun des peptides précités.

Ces résultats, illustrés à la figure 7 (figure 7a : sérums de chats infectés expérimentalement avec du VIF ; figure 7b : sérums de chats infectés naturellement par du VIF), comme précisé ci-dessus, montrent en particulier qu'un test ELISA dont le réactif est le peptide P237 est hautement spécifique (pas de faux positif) et sensible (tous les sérums de chats infectés de différentes origines sont réactifs). De plus, l'utilisation d'un tel peptide synthétique fournit un réactif pur et peu coûteux pour le diagnostic en routine de l'infection à VIF.

En outre, l'absorbance moyenne M des sérums positifs est supérieure pour P237 par rapport aux autres peptides (M₂₃₇ = 1,042 ± 0,110 ; M₂₄₀ = 0,434 ± 0,219 ; M₂₄₁ = 0,660 ± 0,161 ; M₂₄₂ = 0,589 ± 0,136).

Les résultats ci-après montrent également l'intérêt du peptide P237 en tant que réactif dans un test immunodiagnostic :

110 sérums de chats infectés naturellement, 61 sérums de chats infectés expérimentalement avec différents isolats de VIF (Tableau IV) et 61 sérums de chats SPF sont testés par ELISA.

La valeur limite de cet essai est 0,346 (P = 0,088 + 3 x 0,086).

Les 171 sérums de chats infectés par le VIF montrent une réaction positive, alors qu'aucun des chats SPF ne présente de réaction positive.

La figure 8 illustre ces résultats (▲ : chats SPF ; • : chats infectés expérimentalement ; ◆ : chats infectés naturellement).

**TABLEAU IV**

| Chats | Source(s) | Echantillons testés |
|---|---|---|
| SPF | France¹ | 56 |
| | Suisse² | 5 |
| | | |
| infectés naturellement avec VIF | France¹ | 89 |
| | Francec³ | 9 |
| | Suisse² | 7 |
| | Ecosse⁴ | 5 |
| | | |
| inoculés expérimentalement avec des isolats de VIF : | | |
| Wo | France¹ | 22 |
| Me | France¹ | 11 |
| Be | France¹ | 8 |
| Le | France¹ | 7 |
| Br | France¹ | 3 |
| Gi | France¹ | 2 |
| Villefranche | France³ | 1 |
| Envnip | France³ | 1 |
| Petaluma | France³ | 4 |
| Aebli | Suisse² | 1 |
| Gasser | Suisse² | 1 |

| | | |
|---|---|---|
| ¹ Ecole vétérinaire de Maisons-Alfort | | |
| ² Université de Zurich | | |
| ³ Institut Pasteur | | |
| ⁴ Université de Glasgow | | |

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

## Revendications

1. Peptide issu de la protéine Env du VIF de souche Wo, qui constitue un épitope conservé des protéines du VIF, **caractérisé en ce qu'**il est choisi dans le groupe constitué :
- du fragment de 45 aminoacides, dénommé TM3, et correspondant aux positions 744-788 de la séquence de formule I ;
- du fragment de séquence Gln⁷⁶³-Gln-Leu-Gln-Glu-Trp-Glu-Asp⁷⁷⁰ (peptide dénommé P241), correspondant aux positions 763-770 de la séquence de formule I ; et
- du fragment de séquence Val⁷⁷²-Gly-Trp-Ile-Gly-Asn-Ile-Pro⁷⁷⁹ (peptide dénommé P242), correspondant aux positions 772-779 de la séquence de formule I.

2. Vecteur recombinant de clonage et/ou d'expression, **caractérisé en ce qu'**il comprend une séquence nucléotidique codant pour le peptide selon la revendication 1.

3. Vecteur selon la revendication 2, **caractérisé en ce qu'**il est constitué par un plasmide comprenant une origine de réplication, au moins un marqueur de sélection et une séquence nucléotidique codant pour le peptide selon la revendication 1, lequel vecteur est un vecteur de clonage.

4. Vecteur selon la revendication 3, **caractérisé en ce que** ledit vecteur de clonage est constitué par un plasmide capable de se répliquer dans *E. coli*, dans lequel est insérée une séquence nucléotidique codant pour le peptide selon la revendication 1.

5. Vecteur selon la revendication 4, **caractérisé en ce qu'**il est constitué par un vecteur recombinant comprenant une origine de réplication dans un microorganisme hôte approprié, notamment une bactérie ou une cellule eucaryote, au moins un gène dont l'expression permet la sélection soit des bactéries, soit des cellules eucaryotes ayant reçu ledit vecteur, un promoteur permettant l'expression des gènes dans lesdites bactéries ou cellules eucaryotes, et dans lequel est insérée une séquence nucléotidique codant pour le peptide selon la revendication 1.

6. Vecteur selon la revendication 5, **caractérisé en ce qu'**il est constitué par un phage λgt11 dans lequel est inséré, en phase au niveau de son site EcoRI, des fragments de digestion à la DNAse du plasmide pKSe1.

7. Composition à pouvoir immunogène et/ou protecteur, **caractérisée en ce qu'**elle comprend au moins un peptide selon la revendication 1, éventuellement associé à au moins une autre substance immunogène ou immunostimulante et/ou un véhicule pharmaceutiquement acceptable.

8. Procédé de détection d'anticorps anti-VIF, **caractérisé en ce qu'**il consiste à détecter les anticorps anti-VIF éventuellement présents dans un échantillon biologique à l'aide d'un peptide selon la revendication 1, éventuellement fixé sur un support solide approprié, en mettant en présence ledit échantillon biologique avec ledit/lesdits peptides, auxquels se lient les anticorps anti-VIF si de tels anticorps sont présents dans l'échantillon à analyser, la lecture du résultat étant révélée par un moyen approprié, notamment EIA, RIA, fluorescence.

9. Kit, prêt à l'emploi, pour la mise en oeuvre du procédé de détection d'anticorps anti-VIF selon la revendication 8, **caractérisé en ce qu'**il comprend, outre des quantités utiles de tampons appropriés pour la mise en oeuvre de ladite détection, des doses appropriées d'au moins un peptide selon la revendication 1.

10. Réactif de diagnostic de l'immunodéficience féline, **caractérisé en ce qu'**il est choisi dans le groupe constitué par l'un des peptides selon la revendication 1, et **en ce qu'**il constitue un réactif de diagnostic universel de souche de VIF.

## Patentansprüche

1. Peptid, das vom Env-Protein des VIF des Stamms Wo stammt, das ein konserviertes Epitop der Proteine des VIF bildet, **dadurch gekennzeichnet, dass** es aus der Gruppe, bestehend aus
- dem Fragment aus 45 Aminosäuren, das TM3 genannt wird, und den Positionen 744 bis 788 der Sequenz der Formel I entspricht,
- dem Fragment der Sequenz Gln⁷⁶³-Gln-Leu-Gln-Glu-Trp-Glu-Asp⁷⁷⁰ (Peptid, das P241 genannt wird), das den Positionen 763-770 der Sequenz der Formel I entspricht, und
- dem Fragment der Sequenz Val⁷⁷²-Gly-Trp-Ile-Gly-Asn-Ile-Pro⁷⁷⁹ (Peptid, das P242 genannt wird), das den Positionen 772-779 der Sequenz der Formel I entspricht, ausgewählt ist.

2. Rekombinanter Klonierungs- und/oder Expressionsvektor, **dadurch gekennzeichnet, dass** er eine Nukleotidsequenz umfasst, die für das Peptid nach Anspruch 1 kodiert.

3. Vektor nach Anspruch 2, **dadurch gekennzeichnet, dass** er aus einem Plasmid, das einen Replikationsursprung, mindestens einen Selektionsmarker und eine Nukleotidsequenz, die für das Peptid nach Anspruch 1 kodiert, gebildet wird, wobei der Vektor ein Klonierungsvektor ist.

4. Vektor nach Anspruch 3, **dadurch gekennzeichnet, dass** der Klonierungsvektor durch ein Plasmid gebildet wird, das fähig ist, sich in *E. coli* zu replizieren, in den eine Nukleotidsequenz, die für das Peptid nach Anspruch 1 kodiert, insertiert ist.

5. Vektor nach Anspruch 4, **dadurch gekennzeichnet, dass** er durch einen rekombinanten Vektor in einem geeigneten Wirtsmikroorganismus, insbesondere einem Bakterium oder einer Eukaryontenzelle, gebildet wird, umfassend einen Replikationsursprung, mindestens ein Gen, dessen Expression die Selektion der Bakterien oder der Eukaryontenzellen, die den Vektor aufgenommen haben, erlaubt, einen Promotor, der die Expression der Gene in den Bakterien oder Eukaryontenzellen erlaubt, und in den eine Nukleotidsequenz insertiert ist, die für das Peptid nach Anspruch 1 kodiert.

6. Vektor nach Anspruch 5, **dadurch gekennzeichnet, dass** er durch einen Phagen λgt11 gebildet wird, in den Abbaufragmente des Plasmids pKSe1 im Leserahmen auf dem Niveau seiner EcoRI-Schnittstelle insertiert sind.

7. Zusammensetzung mit immunogener Wirkung und/oder Schutzwirkung, **dadurch gekennzeichnet, dass** sie mindestens ein Peptid nach Anspruch 1, gegebenenfalls in Verbindung mit mindestens einer anderen immunogenen oder immunstimulierenden Substanz und/oder ein pharmazeutisch annehmbares Vehikel umfasst.

8. Verfahren zum Nachweis von Antikörpern gegen VIF, **dadurch gekennzeichnet, dass** es darin besteht, Antikörper gegen VIF, die gegebenenfalls in einer biologischen Probe vorliegen, mit Hilfe eines Peptids gemäß Anspruch 1, das gegebenenfalls an einem geeigneten festen Träger fixiert ist, nachzuweisen, wobei die biologische Probe mit dem Peptid/den Peptiden, an das/an die sich die Antikörper gegen VIF binden, wenn solche Antikörper in der zu analysierenden Probe vorliegen, in Kontakt gebracht wird und das Ablesen des Resultats durch ein geeignetes Mittel, insbesondere DIA, RIA, Fluoreszenz, sichtbar gemacht wird.

9. Kit, der zur Verwendung im Verfahren zum Nachweisen von Antikörpern gegen VIF nach Anspruch 8 gebrauchsfertig ist, **dadurch gekennzeichnet, dass** er außer nützlichen Mengen an Puffern, die zur Durchführung des genannten Nachweises geeignet sind, geeignete Dosen mindestens eines Peptids nach Anspruch 1 umfasst.

10. Reagens zur Diagnose von Katzen-Immundefizienz, **dadurch gekennzeichnet, dass** es aus der Gruppe, bestehend aus einem der Peptide gemäß Anspruch 1 ausgewählt ist, und dass es ein universelles Diagnosereagens für einen VIF-Stamm ist.

## Claims

1. Peptide derived from the Env protein of FIV of strain Wo, which constitutes a conserved epitope of the FIV proteins, **characterised in that** it is selected from the group constituted:
- by the 45 amino acid fragment, called TM3, corresponding to positions 744-788 of the sequence of formula I;
- by the fragment having the sequence Gln⁷⁶³-Gln-Leu-Gln-Glu-Trp-Glu-Asp⁷⁷⁰ (peptide called P241), corresponding to positions 763-770 of the sequence of formula I; and
- by the fragment having the sequence Val⁷⁷²-Gly-Trp-Ile-Gly-Asn-Ile-Pro⁷⁷⁹ (peptide called P242), corresponding to positions 772-779 of the sequence of formula I.

2. Recombinant cloning and/or expression vector, **characterised in that** it comprises a nucleotide sequence encoding the peptide according to claim 1.

3. Vector according to claim 2, **characterised in that** it is constituted by a plasmid comprising an origin of replication, at least one selectable marker and a nucleotide sequence encoding the peptide according to claim 1, which vector is a cloning vector.

4. Vector according to claim 3, **characterised in that** the cloning vector is constituted by a plasmid which is capable of replicating itself in E. coli and in which a nucleotide sequence encoding the peptide according to claim 1 is inserted.

5. Vector according to claim 4, **characterised in that** it is constituted by a recombinant vector comprising an origin of replication in a suitable host micro-organism, especially a bacterium or a eukaryotic cell, at least one gene whose expression permits the selection either of the bacteria or of the eukaryotic cells that have received the vector, a promoter permitting the expression of the genes in the bacteria or eukaryotic cells, and in which vector a nucleotide sequence encoding the peptide according to claim 1 is inserted.

6. Vector according to claim 5, **characterised in that** it is constituted by a λgt11 phage in which is inserted, in frame at its EcoRI site, fragments for the DNase digestion of the plasmid pKSe1.

7. Composition having an immunogenic and/or protective power, **characterised in that** it comprises at least one peptide according to claim 1, optionally associated with at least one other immunogenic or immunostimulant substance and/or a pharmaceutically acceptable vehicle.

8. Process for detecting anti-FIV antibodies, **characterised in that** it consists in detecting any anti-FIV antibodies present in a biological sample using a peptide according to claim 1, optionally fixed on a suitable solid support, by bringing together the biological sample and the peptide(s) to which the anti-FIV antibodies bind if such antibodies are present in the sample to be analysed, the reading of the result being revealed by a suitable means, especially EIA, RIA, fluorescence.

9. Ready-for-use kit for implementing the process for detecting anti-FIV antibodies according to claim 8, **characterised in that** it comprises, in addition to useful amounts of suitable buffers for implementing the detection, suitable doses of at least one peptide according to claim 1.

10. Diagnostic reagent for feline immunodeficiency, **characterised in that** it is selected from the group constituted by one of the peptides according to claim 1, and **in that** it constitutes a universal diagnostic reagent for FIV strains.
